# EUROPEAN PATENT APPLICATION

(11) **EP 4 595 983 A1**
(43) Date of publication of application: **06.08.2025**
(21) Application number: 24155814.7
(22) Date of filing: 05.02.2024
(51) Int. Cl.: A61K 47/69, A61K 41/00, A61K 47/54, A61K 49/04, A61P 35/00, C07K 7/08

(54) **NANOPARTICLES FUNCTIONALISED WITH A STABILISING AGENT AND A TARGETING AGENT**

(71) Applicant: BXTA Nanotherapy Limited, Burnham SL1 8DF (GB)
(72) Inventor: Coulter, Jonathan, Burnham, SL1 8DF (GB); Owen, Natalie, Burnham, SL1 8DF (GB); Byrne, Niall, Burnham, SL1 8DF (GB); Dixon, Dorian, Burnham, SL1 8DF (GB)
(74) Representative: J A Kemp LLP

(57) **Abstract**

The present invention relates to functionalised nanoparticles. The present invention also relates to compositions comprising the functionalised nanoparticles, kits, processes for preparing nanoparticles, methods of treating cancer (including prostate cancer), imaging methods, diagnostic methods, methods for killing or attenuating the growth of a cancer cell *in vitro,* nanoparticles and compositions for use in the treatment of cancer or diagnosis, and a novel pepducin.

## Description

### FIELD OF THE INVENTION

The present invention relates to functionalised nanoparticles. The present invention also relates to compositions comprising the functionalised nanoparticles, kits, processes for preparing nanoparticles, methods of treating cancer (including prostate cancer), imaging methods, diagnostic methods, methods for killing or attenuating the growth of a cancer cell *in vitro,* nanoparticles and compositions for use in the treatment of cancer or diagnosis, and a novel pepducin.

### BACKGROUND TO THE INVENTION

Prostate cancer is the most common cancer in men. In the UK alone, more than 48,000 men are diagnosed with prostate cancer annually and there are more than 11,000 deaths annually due to prostate cancer (https://www.cancerresearchuk.org/health-professional/cancer-statistics/statistics-by-cancer-type/prostate-cancer).

*In silico* analysis of publicly available prostate cancer datasets has shown that PTEN^{LOW} and CXCR1/2^{HIGH} tumours are associated with poor prognosis (MSKCC radical prostatectomy cohort & TCGA dataset). The FASTMAN retrospective RT patient cohort sample showed that PTEN^{LOW} and CXCR1/2^{HIGH} tumours are associated with reduced time to BCR and development of metastasis after radiotherapy.

Existing treatments for prostate cancer suffer from a number of problems. For instance, whilst radiotherapy is conventionally seen as the best chance for a 'cure' when treating prostate cancer, major drawbacks include damage surrounding normal/healthy tissue - which occurs in roughly 50% of men (Wolff, R. F. et al. A systematic review of randomised controlled trials of radiotherapy for localised prostate cancer. Eur J Cancer 51, 2345-2367, doi:10.1016/j.ejca.2015.07.019 (2015)) - and recurrence - which occurs in 20 to 30% of men (~3500 men in UK; Dearnaley, D. P. et al. Escalated-dose versus control-dose conformal radiotherapy for prostate cancer: long-term results from the MRC RT01 randomised controlled trial. Lancet Oncol 15, 464-473, doi:10.1016/s1470-2045(14)70040-3 (2014)).

One approach to improve treatment efficacy is the use of nanoparticles (US6955639 B2; US10391174 B2; US8845507 B2; US9433800 B2). However, achieving sufficient target cell nanoparticle uptake represents a major barrier to clinical translation. For instance, the majority of radiosensitising gold nanoparticle studies rely on treatment up to 1 wt.% gold nanoparticle to tumour. The practical implications of upscaling to these concentrations for clinical use would prove prohibitive on a cost and potentially toxicity basis. Furthermore, `as-synthesised' nanoparticles often aggregate at physiological conditions, significantly reducing efficacy / cellular uptake / radiosensitisation. To seek to address this stability issue, polyethylene glycol (PEG) has been used as a stabiliser (US8033977 B2; US9486480 B2). However, PEG severely hinders internalisation. There therefore remains a need to provide an effective treatment for cancer, in particularly prostate cancer, that enjoys improved efficacy and reduced harmful side effects, especially those caused by radiation.

All references mentioned herein are incorporated by reference in their entirety.

### SUMMARY OF THE INVENTION

The present inventors have now surprisingly found that the present invention provides a nanoparticle that enjoys robust stability and excellent cellular uptake. In particular, the nanoparticles of the invention enjoy one or more of the following advantages: inhibition of pro-survival inflammatory signalling, exploitation of radiation induced inflammation as a target, increased cellular uptake, increased target specificity, low cytotoxicity, increased radiosensitisation (including at clinical energies), increased image enhancement, or a combination thereof. A significant improvement on current radiotherapy efficacy is therefore provided. Similarly, the use of lower doses of radiation in radiotherapy is enabled, thereby reducing the damage to healthy cells and harmful side effects to patients / subjects being treated.

The present invention relates to a nanoparticle functionalised with a stabilising agent and a targeting agent. The present invention also relates to compositions comprising nanoparticles, kits comprising nanoparticles, a pepducin, processes for preparing nanoparticles, methods of treating cancer (including prostate cancer), imaging methods, diagnostic methods, and methods for killing or attenuating the growth of a cancer cell *in vitro.*

The present invention provides the following:
[1] A nanoparticle comprising a core, wherein the core preferably comprises a chemical element having an atomic number of from about 44 to about 83, and wherein the core is functionalised with a stabilising agent and a targeting agent. The presence of both stabilising and targeting agents results in effective nanoparticle stability whilst simultaneously maintaining good cellular uptake.
[2] The nanoparticle of [1], wherein the targeting agent is not RME peptide (CKKKKKKSEDEYPYVPN) or H5WYG peptide (GCGLFHAIAHFIHGGWHGHHGWYG).
[3] The nanoparticle of [1] or [2], wherein the targeting agent comprises an arginine residue.
[4] The nanoparticle of any one of [1] to [3], wherein the targeting agent comprises a peptide chain having between 3 and 15 amino acid residues or between 20 to 50 amino acid residues.
[5] The nanoparticle of any one of [1] to [4], wherein the nanoparticle is for treating cancer, preferably prostate cancer.
[6] The nanoparticle of any one of [1] to [5], wherein:
   (i) the core comprises a chemical element selected from gold, platinum, iridium, osmium, bismuth, tantalum, thulium, hafnium and gadolinium, optionally wherein the core consists of gold, platinum, iridium, osmium, an oxide of bismuth, an oxide of tantalum, an oxide of thulium, an oxide of hafnium, or an oxide of gadolinium; and/or
   (ii) the core comprises elemental gold, optionally wherein the core consists of elemental gold; and/or
   (iii) the core is a solid core, optionally wherein the core is a solid core that is spherical or approximately spherical in shape; and/or
   (iv) the core comprises an additional chemical element in addition to the chemical element having an atomic number of from 44 to about 83, for instance a magnetic chemical element such as iron.
   These specific core materials are particularly biocompatible and pose a minimal risk (if any) to normal tissue. These materials also provide significant radiosensitising effects. The presence of a magnetic chemical element can be used to imbue the nanoparticle with magnetic properties, which can aid systemic delivery and targeting.
[7] The nanoparticle of any one of [1] to [6], wherein the core has a mean hydrodynamic diameter of from about 5 to about 50 nm. Nanoparticles of this size enjoy good stability and cellular uptake.
[8] The nanoparticle of [7], wherein the mean hydrodynamic diameter is determined using dynamic light scattering in accordance with ISO 22412:2017.
[9] The nanoparticle of any one of [1] to [8], wherein the nanoparticle is obtainable by combining the core with:
   (i) the stabilising agent and the targeting agent, or
   (ii) a stabilising agent precursor and the targeting agent, or
   (iii) the stabilising agent and a targeting agent precursor, or
   (iv) a stabilising agent precursor and a targeting agent precursor,
   wherein the stabilising agent, or precursor thereof, and the targeting agent, or precursor thereof, are added respectively at a molar ratio of from about 1:2 to about 1:5, optionally from about 1:2 to about 1:4. Nanoparticles having this ratio of stabilising agent to targeting agent have particularly good stability and cell internalisation, with a 1:3 ratio typically being optimal.
[10] The nanoparticle of any one of [1] to [9], wherein the stabilising agent and targeting agent are independently attached to the core via a coordinate covalent bond, a covalent bond, or an ionic bond. When the stabilising and targeting agents are connected to the nanoparticle via these interactions, the connection is generally robust and detachment of functional groups, e.g. during use, and associated loss of efficacy is generally minimised or completely prevented.
[11] The nanoparticle of any one of [1] to [10], wherein the stabilising agent enhances the stability of the nanoparticle, optionally wherein the stabilising agent reduces agglomeration and deterioration, especially under biological or physiological conditions. The enhanced stability provided by the stabilising agent enables superior nanoparticle efficacy, as explained above.
[12] The nanoparticle of any one of [1] to [11], wherein the stabilising agent comprises a natural polymer, synthetic polymer, biocompatible polymer, biomedical polymer, hydrophilic polymer, or a combination thereof, optionally wherein the natural polymer, synthetic polymer, biocompatible polymer, biomedical polymer, and/or hydrophilic polymer is attached to the core via a coordinate covalent bond. These stabilising agents are advantageous, and the coordinate covalent bond is a particularly strong and advantageous attachment interaction.
[13] The nanoparticle of any one of [1] to [12], wherein the stabilising agent comprises polyethylene glycol, polyethyleneimine, poly(N-(2-hydroxypropyl) methacrylamide, chitosan, or a combination thereof, optionally wherein the polyethylene glycol, polyethyleneimine, poly(N-(2-hydroxypropyl) methacrylamide, and/or chitosan is attached to the core via a coordinate covalent bond. These stabilising agents are particularly advantageous, and provide excellent nanoparticle stabilisation.
[14] The nanoparticle of any one of [1] to [13], wherein the stabilising agent comprises polyethylene glycol, and/or has a weight average molecular weight of from about 800 to about 10000 g/mol, preferably from about 3000 to about 7000 g/mol. PEG is also particularly advantageous from a stability perspective, and is known to improve biocompatibility, reduce opsonisation by proteins and impair nanoparticle uptake by macrophages, preventing the formation of a protein-corona. PEGs of these molecular weights are particularly advantageous and provide the required stability whilst minimising negative impacts on endocytosis.
[15] The nanoparticle of any one of [1] to [14], wherein the stabilising agent is thiol-terminated. The presence of an S atom in the thiol termination enables an advantageous attachment interaction to the core, for instance an Au-S co-ordinate bond for gold nanoparticles.
[16] The nanoparticle of any one of [1] to [15], wherein the targeting agent targets cells expressing chemokine receptors, optionally wherein (i) the targeting agent enables cellular uptake of the nanoparticle in cells expressing chemokine receptors, and/or (ii) the chemokine receptors are selected from CXCR1, CXCR2, and combinations thereof. C12 - exploits the tumour stress response to radiotherapy to improve nanoparticle internalisation into the tumour. A typical EBRT radiotherapy treatment plan delivers 60 Gy in twenty individual 3 Gy fractions. Doses in this range have been proven to enhance the tumour expression of CXCR1/2, therefore RT will act to prime the tumour to overexpress the targeting receptor for the nanoparticles.
[17] The nanoparticle of any one of [1] to [16], wherein the targeting agent comprises a peptide and/or small molecule, optionally wherein:
   (i) the peptide is a pepducin, protein, polypeptide, oligopeptide, aptamer, antibody, and/or antibody fragment, and/or
   (ii) the small molecule is a small molecule inhibitor, such as a small molecule inhibitor that targets CXCR1 or CXCR2.
[18] The nanoparticle of any one of [1] to [17], wherein the targeting agent comprises a peptide, preferably a pepducin or a protein, optionally wherein the targeting agent is a peptide. The targeting agents of [17] and [18] are particularly advantageous targeting agents, and provide excellent cellular uptake.
[19] The nanoparticle of any one of [1] to [18], wherein the targeting agent comprises a pepducin comprising a peptide chain and an N-terminal lipid moiety, wherein the peptide chain comprises fewer than about 50 amino acid residues and has a C-terminal cysteine residue, optionally wherein:
   (i) the peptide chain is derived from the amino acid sequence of an intracellular loop domain of a G protein coupled receptor; and/or
   (ii) the peptide chain is derived from the amino acid sequence of a chemokine receptor; and/or
   (iii) the peptide chain is derived from the amino acid sequence of CXCR1 or CXCR2; and/or
   (iv) the peptide chain comprises an amino acid sequence having at least about 80% sequence identity to SEQ ID 1; and/or
   (v) the N-terminal lipid moiety is a C10-25 lipid moiety, preferably a moiety derived from a C10-C25 fatty acid, more preferably a moiety derived from palmitic acid, myristic acid, stearic acid or lithocholic acid, even more preferably a palmitoyl moiety, and/or
   (vi) the C-terminal cysteine residue is amidated.
[20] The nanoparticle of any one of [1] to [19], wherein the targeting agent comprises a pepducin comprising an N-terminal palmitoyl moiety and a C-terminal cysteine residue, wherein the pepducin has a peptide chain having at least about 80% sequence identity to SEQ ID 1, and wherein the pepducin comprises fewer than about 50 amino acid residues, optionally wherein the targeting agent comprises a pepducin corresponding to SEQ ID 2 or SEQ ID 3. The targeting agents of [19] and [20] are particularly advantageous targeting agents, and block CXCR2 signal transduction in addition to improving cellular uptake.
[21] The nanoparticle of any one of [1] to [20], wherein:
   - the core comprises elemental gold,
   - the stabilising agent comprises a polyethylene glycol that is attached to the core via a coordinate covalent bond, and
   - the targeting agent comprises a pepducin that is attached to the core via a coordinate covalent bond.
[22] The nanoparticle of any one of [1] to [21], wherein:
   - the core comprises elemental gold,
   - the stabilising agent comprises a thiol-terminated polyethylene glycol, and
   - the targeting agent comprises a pepducin comprising an N-terminal palmitoyl moiety and a C-terminal cysteine residue, wherein the pepducin has a peptide chain having at least about 80% sequence identity to SEQ ID 1 and the pepducin comprises fewer than about 50 amino acid residues, optionally wherein the targeting agent comprises a pepducin corresponding to SEQ ID 2 or SEQ ID 3.
[23] A nanoparticle consisting of an elemental gold core that is functionalised with a thiol-terminated polyethylene glycol and a pepducin comprising an N-terminal palmitoyl moiety and a C-terminal cysteine residue, wherein the pepducin has a peptide chain having at least about 90% sequence identity to SEQ ID 1 and comprises fewer than about 50 amino acid residues, optionally wherein the pepducin corresponds to SEQ ID 2 or SEQ ID 3. The nanoparticles of [21] to [23] are particularly advantageous nanoparticles, and enjoy inhibition of pro-survival inflammatory signalling, exploitation of radiation induced inflammation as a target, increased cellular uptake, increased target specificity, low cytotoxicity, and increased radiosensitisation (including at clinical energies), increased image enhancement.
[24] A composition comprising one or more nanoparticle(s) according to any one of [1] to [23], optionally wherein the composition is a colloid solution. The composition is beneficial for various applications and facilitates use of the nanoparticles of the invention.
[25] The composition of [24], wherein the composition is a pharmaceutical composition that comprises a therapeutically effective amount of the nanoparticle(s) and a pharmaceutically acceptable excipient. This composition is beneficial for treatment / therapy, especially relating to cancer. In particular, improved efficacy and/or reduced side effects caused by radiation can be achieved.
[26] The composition of [24], wherein the composition is a diagnostic composition. This composition is beneficial for diagnosis / imaging, especially relating to cancer. In particular, enhanced contrast / image quality can be achieved.
[27] The composition of any one of [24] to [26], wherein the concentration of the nanoparticle(s) in the composition is about 20 µg/L to about 200 g/L. This concentration of nanoparticles in the composition provides particularly advantageous results.
[28] A kit comprising:
   - one or more nanoparticle(s) according to any one of [1] to [23] or a composition according to any one of [24] to [27]; and
   - means for administering the nanoparticle(s) or composition to a subject.
   It is convenient to have all the necessary components in a single kit: it facilitates the use of the invention for the user.
[29] A pepducin comprising a peptide chain and an N-terminal lipid moiety derived from a C10-C25 fatty acid, wherein the peptide chain has an N-terminal arginine residue and a C-terminal cysteine residue, and the peptide chain has at least about 90% sequence identity to SEQ ID 1, and wherein the pepducin comprises fewer than about 50 amino acid residues, optionally wherein (i) the C10-C25 fatty acid is selected from palmitic acid, myristic acid, stearic acid and lithocholic acid ; and/or (ii) the pepducin corresponds to SEQ ID 2 or SEQ ID 3; and/or (iii) the pepducin corresponds to SEQ ID 3. This pepducin is a CXCR1/2 antagonist and has been shown to block CXCR2 signal transduction in *in vitro* and *in vivo* cancer models. It both antagonises the CXCR1/2 receptors to switch off pro-survival and pro-tumour signalling while simultaneously promoting nanoparticle internalisation. Further, when used with the nanoparticles of the invention (as the targeting agent), it enables - in combination with the core and stabilising agent - inhibition of pro-survival inflammatory signalling, exploitation of radiation induced inflammation as a target, increased cellular uptake, increased target specificity, low cytotoxicity, increased radiosensitisation (including at clinical energies), increased image enhancement, and combinations thereof.
[30] A process for preparing a nanoparticle, said process comprising:
   (a) combining a metal salt with a reducing agent to obtain a dispersion comprising an unfunctionalised nanoparticle, wherein the metal salt comprises a chemical element having an atomic number of from about 44 to about 83; and
   (b) to the dispersion obtained in step (a), adding a stabilising agent or precursor thereof and a targeting agent or precursor thereof at a molar ratio of from about 1:2 to about 1:5 respectively to obtain a nanoparticle functionalised with both a stabilising agent and a targeting agent;
   optionally wherein the process further comprises dispersing the functionalised nanoparticle obtained in step (b) in water, an aqueous solution or a buffer to create a colloid solution.
[31] The process of [30], wherein the nanoparticle is a nanoparticle according to any one of [1] to [23]. The processes of [30] and [31] provide a synthesis for the nanoparticles of the invention.
[32] A method of treating cancer comprising administering a nanoparticle according to any one of [1] to [23] or a composition according to any one of [24] to [27] to a subject having cancer.
[33] The method of [32], further comprising the use of ionising radiation radiotherapy to irradiate the site of the cancer within the subject following administration of the nanoparticle or composition to the subject, optionally wherein the ionising radiation is X-ray radiation, γ-ray photon beam radiation, electron beam radiation, positron beam radiation, proton beam radiation, radioisotope emission radiation, or a combination thereof.
[34] The method of [33], wherein the ionising radiation is of about 2 kV to about 24 MV, optionally as about 500 kV to about 18 MV.
[35] A method of imaging a subject comprising administering a nanoparticle according to any one of [1] to [23] or a composition according to any one of [24] to [27] to a subject and then imaging the subject using an imaging device.
[36] A nanoparticle according to any one of [1] to [23] or a composition according to any one of [24] to [27] for use in the treatment of cancer, the treatment comprising administering the nanoparticle or composition to a subject having cancer. The methods / nanoparticles and compositions for use of [32] to [36] enjoy the benefits listed above, most notably more efficacious treatment and/or reduced harmful side-effects caused by radiation.
[37] A nanoparticle according to any one of [1] to [23] or a composition according to one of [24] to [27] for use in a method of diagnosis, optionally wherein the method comprises detecting the presence of cancer in a patient. This nanoparticle / composition for use enables improved CT contrast and provides an efficient contrast agent. It can also improve prostate tumour delineation on CT.
[37] An *in vitro* method for killing or attenuating the growth of a cancer cell, the method comprising:
   - treating the cancer cell with a nanoparticle according to any one of [1] to [23] or a composition according to [24], [25] or [27], and then
   - irradiating the cancer cell with ionising radiation.
   Such a method may, for instance, be useful for studying cancer cells and in the development of future cancer treatments.
[39] The method of any one of [32] to [34] or [38] or the nanoparticle or composition for use of [36] or [37], wherein the cancer is selected from prostate cancer, breast cancer and/or lung cancer, preferably wherein the cancer is prostate cancer. The benefits of the invention are especially applicable to these cancers, and prostate cancer in particular.

### BRIEF DESCRIPTION OF THE FIGURES

The following section provides a brief description of the Figures. Additional commentary is provided in the detailed description and Examples section. Note that some Figures refer to the pepducin of the invention corresponding to SEQ ID 3 as X1/2pal-i3+C, and the corresponding pepducin lacking the C-terminal cysteine residue as X1/2pal-i3.

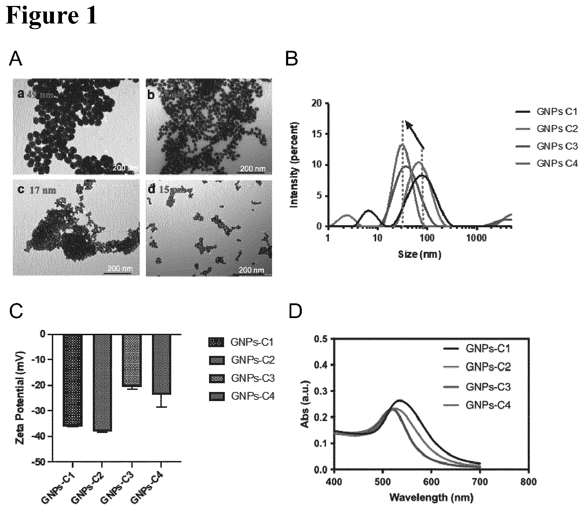

Figure 1: physiochemical characterisation of functionalised nanoparticle formulation (corresponding to the formulation prepared in Example 1); (a) transmission electron microscopy (TEM) of nanoparticles prepared using different amount of sodium citrate (a. 0.27 mM - C1; b. 0.54 mM - C2; c. 0.82 mM - C3; d. 1.28 mM - C4); (b) hydrodynamic size, and (c) zeta potential (charge) of nanoparticle preparations as determined by dynamic light scattering (DLS) using Malvern Zetasizer: nanoparticles C1 (49 nm), C2 (29nm), C3 (17nm), and C4 (15nm); (d) UV/Vis spectra of nanoparticle preparations.

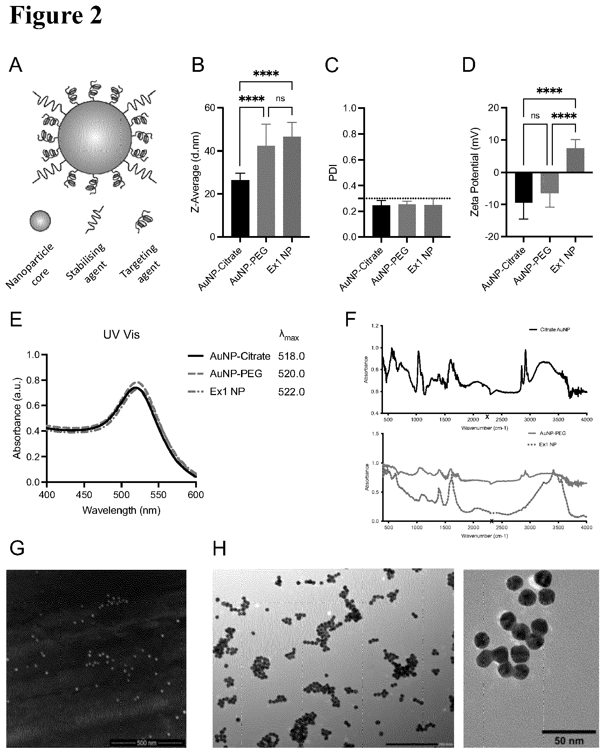

Figure 2: physiochemical characterisation of functionalised nanoparticle formulation (corresponding to the formulation prepared in Example 1); (a) schematic of nanoparticle functionalised with stabilising agent and targeting agent; (b) hydrodynamic size, (c) polydispersity index (PDI) and (d) zeta potential (charge) of nanoparticle preparations as determined by dynamic light scattering (DLS) using Malvern Zetasizer (values are shown for independent batch preparations of nanoparticles, and bars indicate mean with SD; n=3); (e) UV/Vis spectra of nanoparticle preparations; λ max (nm) for nanoparticles in parentheses; (f) Fourier transform infrared spectroscopy (FTIR) of AuNP-citrate (top), AuNP-PEG and inventive (bottom) nanoparticle preparations; (g) scanning electron microscopy (SEM); (h) transmission electron microscopy (TEM) of nanoparticle preparations.

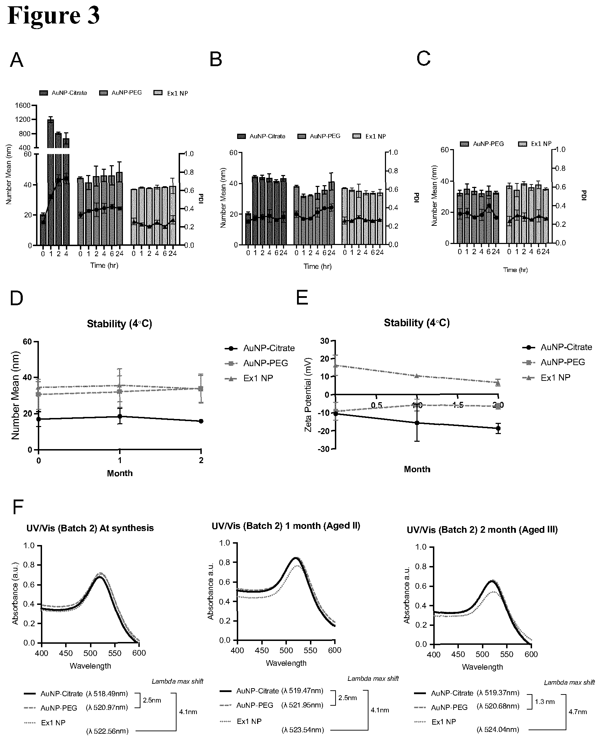

Figure 3: stability of nanoparticle functionalised formulation an inventive nanoparticle formulation (corresponding to the formulation prepared in Example 1); hydrodynamic size (bars, +/- SD) and PDI (lines, +/- SD) of nanoparticle preparations as determined by DLS over time following exposure to (a) 0.15 M NaCl, (b) 10% FCS or (c) 40% FCS (n=3; statistical significance determined by one-way ANOVA with Dunnett multiple comparison test); (d) hydrodynamic size, (e) zeta potential; (f) UV/Vis spectra of nanoparticle preparations over 2 months (4 °C).

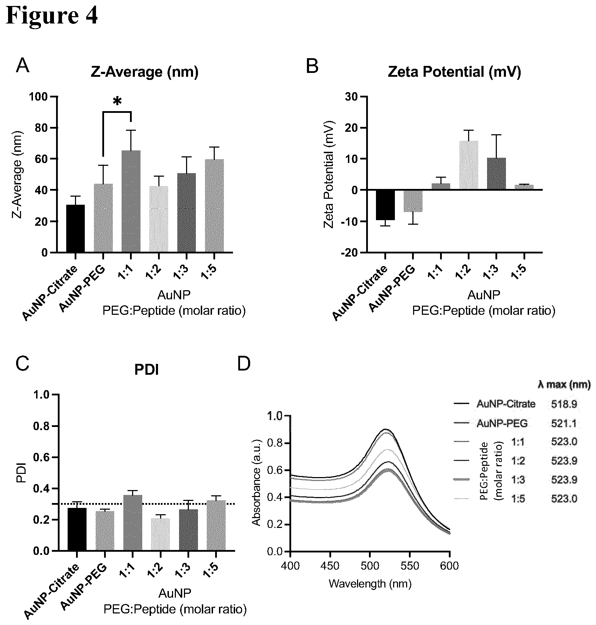

Figure 4: functionalised nanoparticle formulations synthesised with alternative stabilising agent to targeting agent molar ratios. The core was gold, the stabilising agent was PEG, and the targeting agent was the pepducin corresponding to SEQ ID 3. Comparison of 1:1, 1:2, 1:3 and 1:5 molar ratios of stabilising agent to targeting agent gold nanoparticle preparations (a) hydrodynamic size, (b) polydispersity index (PDI) and (c) zeta potential (charge) as determined by dynamic light scattering (DLS) using Malvern Zetasizer (bars indicate mean with SD; n=≥3); (d) UV/Vis spectra of nanoparticle preparations; λ max (nm) in parentheses.

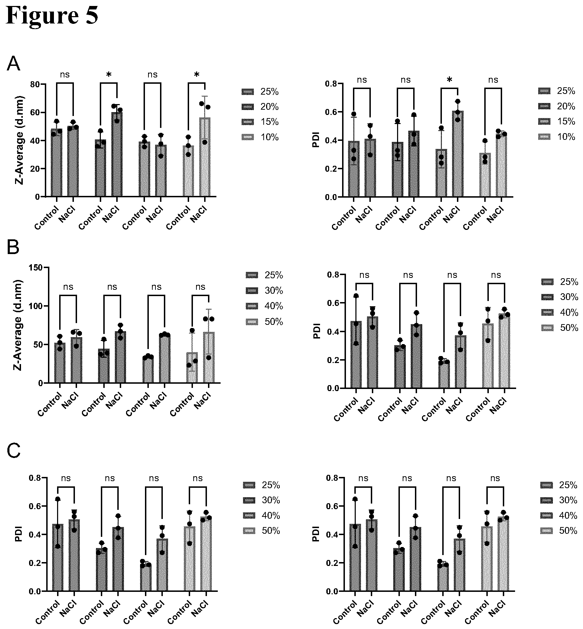

Figure 5: functionalised nanoparticle formulations synthesized with alternative stabilising agent molecular weights. The core was gold, the stabilising agent was PEG. Nanoparticles were functionalised with either (a) 5 kDa, (b) 2 kDa, or (c) 800 Da molecular weight PEG. The figure shows hydrodynamic size (left panel) and polydispersity index (PDI) (right panel) as determined by dynamic light scattering (DLS) using Malvern Zetasizer before and following exposure to 0.15 M NaCl; values are shown for independent batch preparations of nanoparticles (bars indicate mean with SD; n=3).

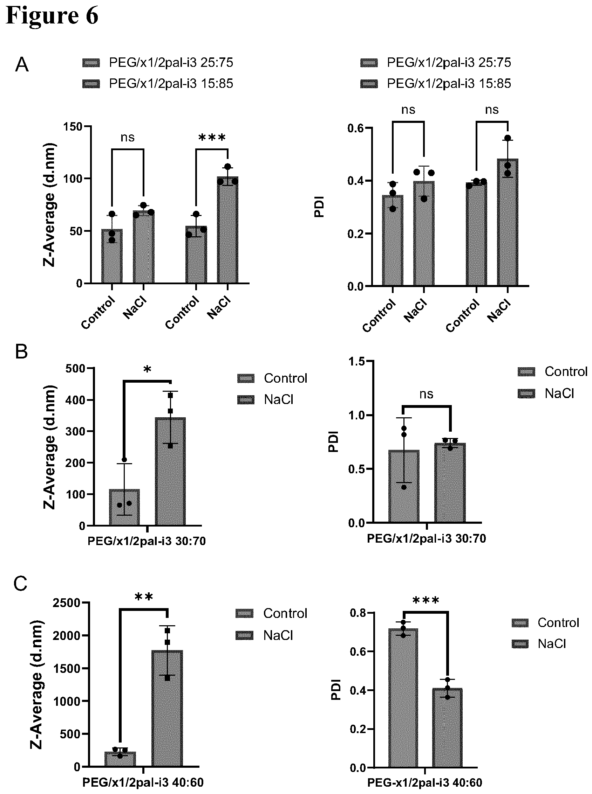

Figure 6: stability of functionalised nanoparticle formulations synthesised with alternative stabilising molecular weights. Nanoparticles were co-functionalised with either (a) 5 kDa, (b) 2 kDa, and (c) 800 Da molecular weight PEG and a targeting agent (pepducin corresponding to SEQ ID 3). The core was gold, the stabilising agent was PEG, and the targeting agent was the pepducin corresponding to SEQ ID 3. The figure shows hydrodynamic size (left panel) and polydispersity index (PDI) (right panel) as determined by dynamic light scattering (DLS) using Malvern Zetasizer before and following exposure to 0.15 M NaCl; values are shown for independent batch preparations of nanoparticles (bars indicate mean with SD; n=3).

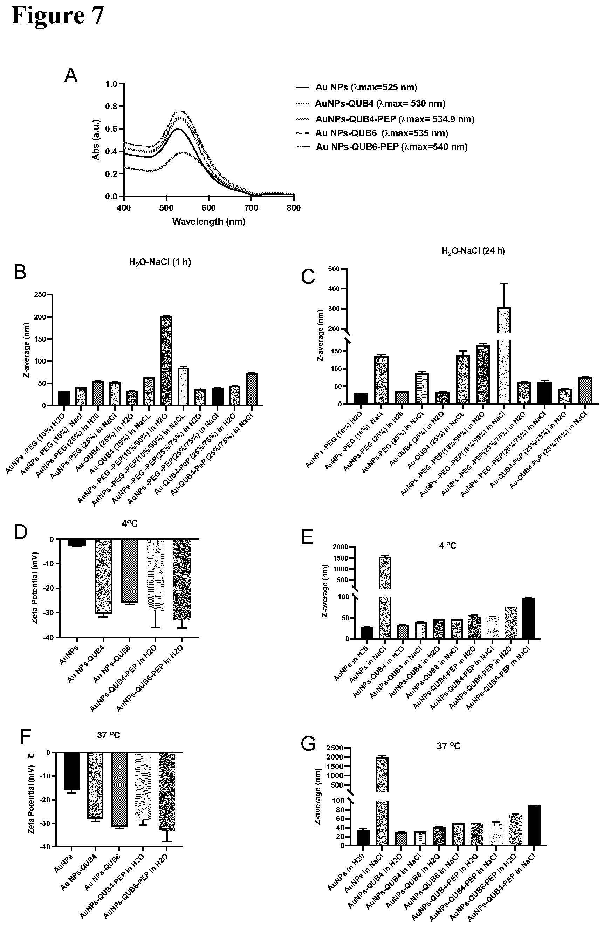

Figure 7: functionalised nanoparticle formulations synthesised with an alternative stabilising agent, pHPMA. Gold nanoparticles were co-functionalised with one of two thiolated HPMA polymers (QUB4 or QUB6) as the stabilising agent and the pepducin corresponding to SEQ ID 3 as the targeting agent. The core was gold. (a) UV/Vis spectra of the nanoparticle preparations. Hydrodynamic size at 1 hr (b) and 24 hr (c) following exposure to 0.15 M NaCl, as determined by DLS; (d, f) zeta potential (charge) and (e, g) hydrodynamic size of nanoparticle preparations after incubation at (d, e) 4 °C and (f, g) 37 °C.

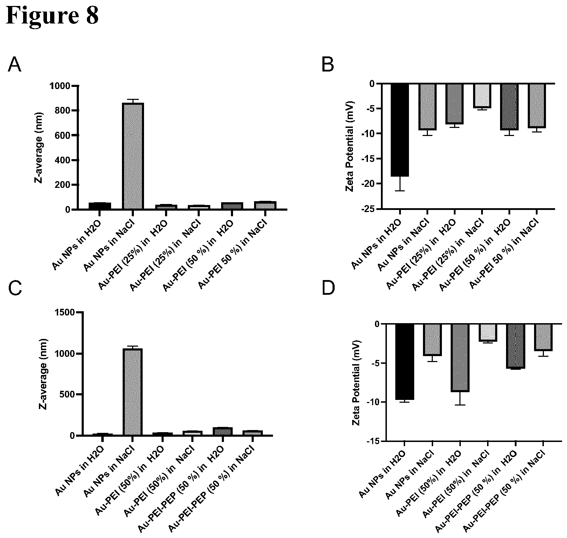

Figure 8: functionalised nanoparticle formulations synthesised with the alternative stabilising agent polyethylenimine (PEI). Gold core nanoparticles were co-functionalised with PEI-G-PEG-SH (PEI, 2 kDa; PEG, 5 kDa) as the stabilising agent and with the pepducin corresponding to SEQ ID 3 as the targeting agent, (a) hydrodynamic size and (b) zeta potential (charge) of the nanoparticle preparations at different PEI nanoparticle surface coverage densities as determined by DLS following exposure to 0.15 M NaCl. (c) hydrodynamic size and (d) zeta potential (charge) of nanoparticle preparations at different PEI surface coverage densities and co-functionalised with pepducin as determined by DLS following exposure to 0.15 M NaCl.

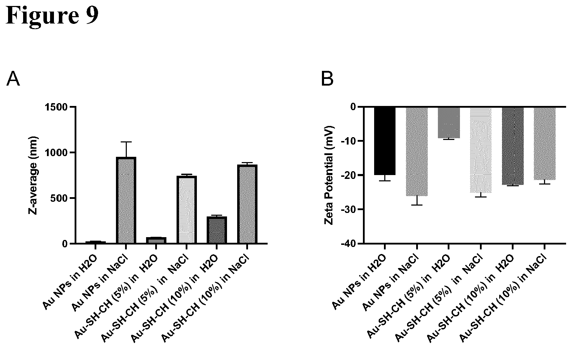

Figure 9: functionalised nanoparticle formulations synthesised with another stabilising agent, chitosan. Gold nanoparticles were functionalised with thiolated chitosan (5 kDa); (a) hydrodynamic size and (b) zeta potential (charge) as determined by dynamic light scattering (DLS) using Malvern Zetasizer before and following exposure to 0.15 M NaCl.

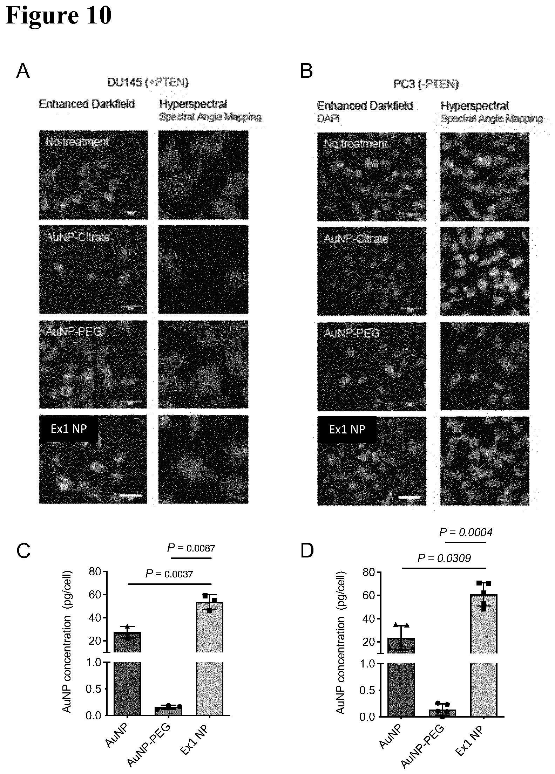

Figure 10: internalisation of functionalised nanoparticle formulation (corresponding to the formulation prepared in Example 1) in prostate cancer cells *in vitro.* Representative darkfield, hyperspectral imaging and spectral angle mapping (SAM) of (a) PTEN-expressing DU145 and (b) PTEN-null PC3 cells following treatment with 100 µg/ml nanoparticles for 24 hr; scale bar 40 µm; (c) quantification of gold internalisation in (c) DU15 and (d) PC3 cells using atomic absorption spectroscopy (AAS); values shown for independent experiments (bars indicate mean with SD; n=3 (DU145), n=5 (PC3)).

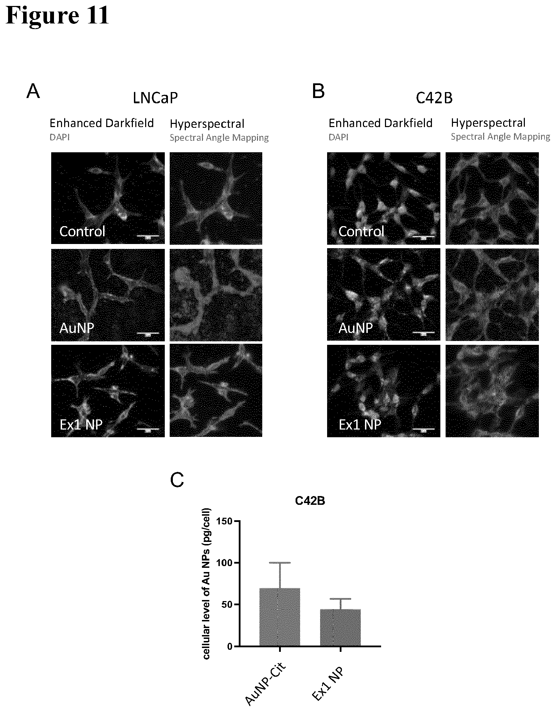

Figure 11: internalisation of functionalised nanoparticle formulation (corresponding to the formulation prepared in Example 1) in prostate cancer cells *in vitro.* Representative darkfield, hyperspectral imaging and spectral angle mapping (SAM) of (a) LNCaP and (b) C42B cells without (control) or following treatment with 100 µg/ml nanoparticles for 24 hr; scale bar 40 µm; (c) quantification of gold internalisation in C42B cells using atomic absorption spectroscopy (AAS); values shown for independent experiments (bars indicate mean with SD; n=3 (C42B)).

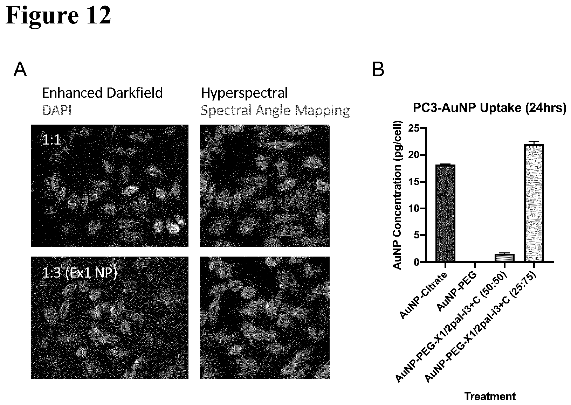

Figure 12: internalisation of functionalised nanoparticle formulations synthesised with alternative stabilising agent to targeting agent molar ratios. The core was gold, the stabilising agent was PEG, and the targeting agent was the pepducin corresponding to SEQ ID 3. (a) representative darkfield, hyperspectral imaging and spectral angle mapping (SAM) of PTEN-null PC3 cells without (control) or following treatment with 100 µg/ml nanoparticles at 1:1 and 1:3 molar ratios of stabilising agent to targeting agent for 24 hr; (b) quantification of gold internalisation in PC3 cells using atomic absorption spectroscopy (AAS); values shown for independent experiments (bars indicate mean with SD; n=2).

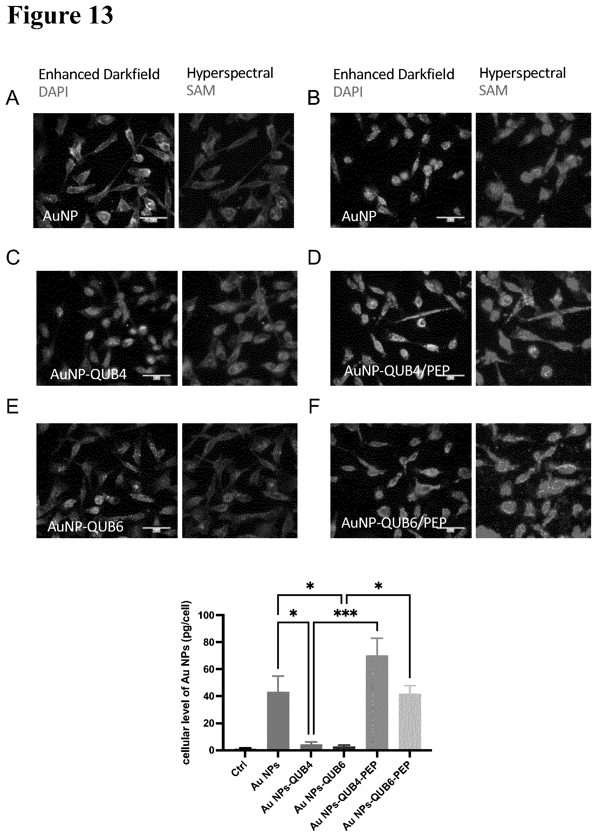

Figure 13: internalisation of functionalised gold nanoparticle formulations synthesised with alternative stabilising agent, pHPMA. Representative darkfield, hyperspectral imaging and spectral angle mapping (SAM) of PTEN-null PC3 cells without (control) or following treatment with (a) control, or (b) AuNP-citrate, (c) AuNP-QUB4, (d) AuNP-QUB4-Pep, (e) AuNP-QUB6, (f) AuNP-QUB6-Pep at 100 µg/ml for 24 hr; scale bar 40 µm; (g) quantification of gold internalisation in PC3 cells using atomic absorption spectroscopy (AAS); values shown for independent experiments (bars indicate mean with SD; n=3).

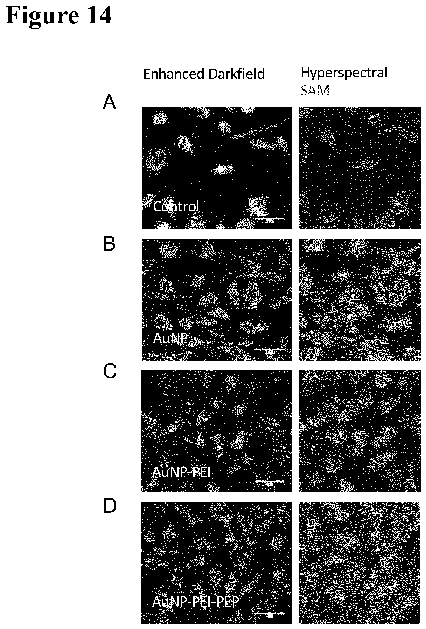

Figure 14: internalisation of functionalised gold nanoparticle formulations synthesised with alternative stabilising agent, PEI. Representative darkfield, hyperspectral imaging and spectral angle mapping (SAM) of PTEN-null PC3 cells without (control) or following treatment with (a) control, or (b) AuNP-citrate, (c) AuNP-PEI, (d) or AuNP-PEI-PEP at 100 µg/ml for 24 hr; scale bar 40 Mm.

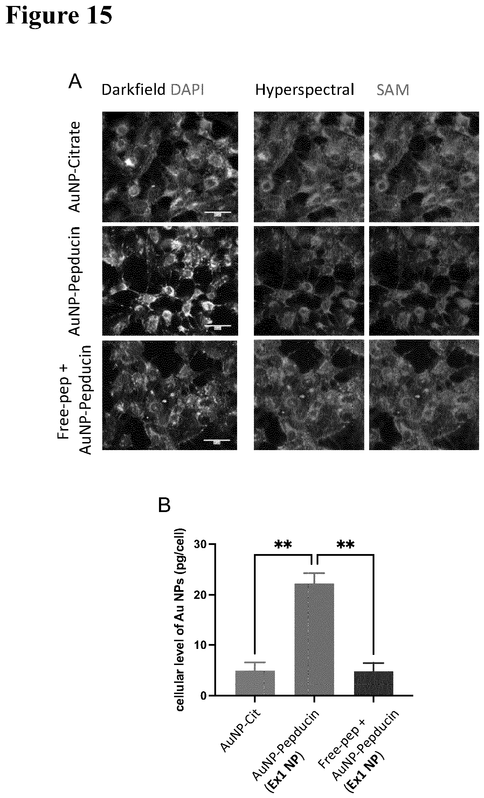

Figure 15: specificity of functionalised nanoparticle formulation (corresponding to the formulation prepared in Example 1) targeting and internalisation in PC3 cells *in vitro;* (a) representative darkfield, hyperspectral imaging and spectral angle mapping (SAM) of PC3 cells following 2 hr treatment with AuNP-citrate, the functionalised nanoparticle formulation or the functionalised nanoparticle formulation pre-treated with free peptide (pepducin corresponding to SEQ ID 3; 600 nM); scale bar 40 µm; (b) quantification of gold internalisation in PC3 cells using AAS (bars indicate mean with SD; n=3).

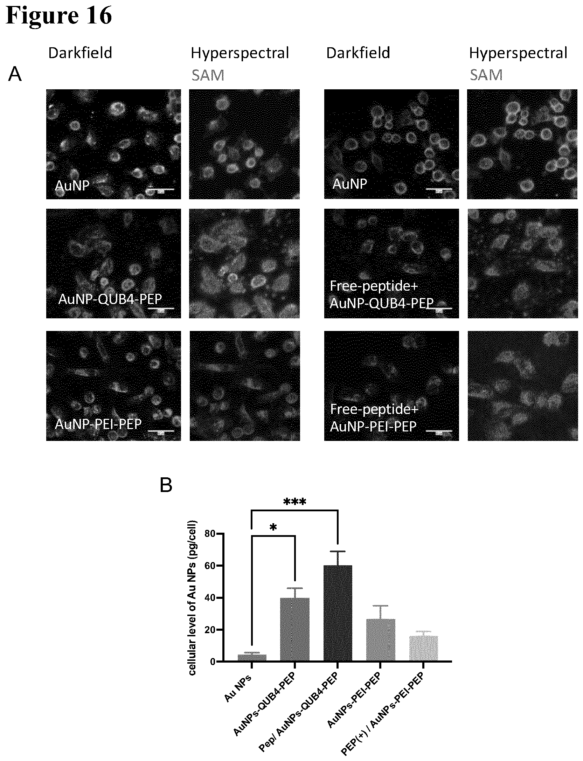

Figure 16: specificity of functionalised nanoparticle formulations in PC3 cells *in vitro.* Representative darkfield, hyperspectral imaging and spectral angle mapping (SAM) of PC3 cells following 2 hr treatment with AuNP-citrate, functionalised nanoparticle formulation corresponding to the formulation prepared in Example 1 (100 µg/ml) or the same inventive formulation pre-treated with free peptide (pepducin corresponding to SEQ ID 3; 600 nM); scale bar 40 µm; (b) quantification of gold internalisation in PC3 cells using AAS; (bars indicate mean with SD; n=3).

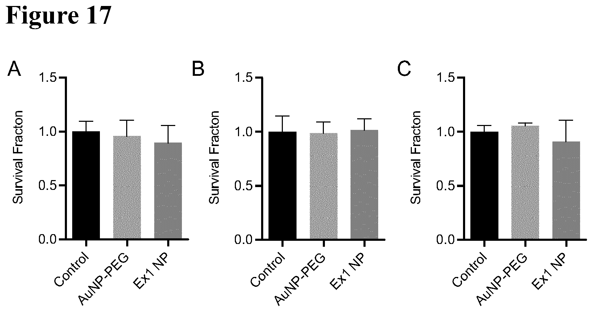

Figure 17: long term cytotoxicity and radiosensitising efficacy of functionalised nanoparticle formulation (corresponding to the formulation prepared in Example 1) in prostate epithelial and cancer cells *in vitro.* Survival fractions of (a) DU145 and (b) PC3 prostate cancer and (c) PNT2 prostate epithelial cells 14-days post treatment with 100 µg/ml of functionalised nanoparticle formulations as determined by clonogenic survival assay (bars indicate mean with SD, n=2 (sh11.02, PNT2), n=3 (DU145, PC3).

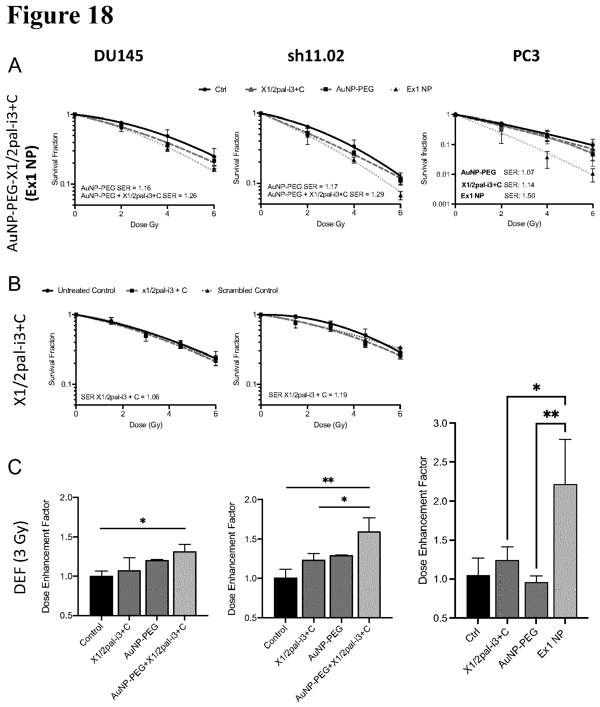

Figure 18: comparison of the radiosensitising efficacy of functionalised nanoparticle formulation (corresponding to the formulation prepared in Example 1) against pepducin corresponding to SEQ ID 3 as a single-agent in prostate cancer cells at kV energies *in vitro.* Cells were treated with or without 100 µg/ml of AuNP-PEG, the functionalised nanoparticle or 800 nM of the free pepducin 24 hrs prior to irradiation (0-6 Gy). Clonogenic survival assays of PTEN-expressing DU145 and PTEN-null sh11.02 and PC3 prostate cancer cells following treatment with (a) the functionalised nanoparticle formulation, and (b) the free pepducin (values indicate mean with SD; n=3; lines represent linear quadratic curve fit from which sensitiser enhancement ratios (SER) were determined, ratio of control to treated; statistical significance determined by two-way ANOVA); (c) comparison of survival fractions at a clinically relevant dose of 3 Gy shows significant increases in dose enhancement (DEF) in prostate cancer cells following treatment with the functionalised nanoparticles. Treatment with the free pepducin as a single-agent did not show such a significant dose enhancement.

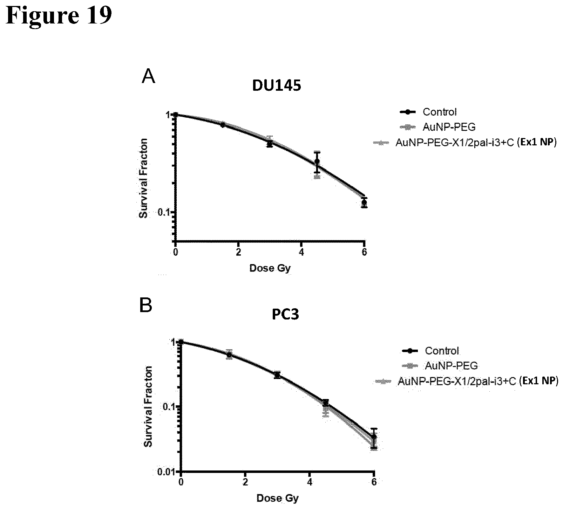

Figure 19: radiosensitising efficacy of functionalised nanoparticle formulations synthesised with alternative stabilising agent to targeting agent molar ratios in prostate cancer cells at kV energies *in vitro.* The core was gold, the stabilising agent was PEG, and the targeting agent was the pepducin corresponding to SEQ ID 3. Cells were treated with or without 150 µg/ml AuNPs at 1:1 molar ratios of stabilising agent to targeting agent for 24 hr prior to irradiation (0 - 6Gy). Clonogenic survival assays show no additional radiosensitisation benefit in PC3 or DU145 prostate cancer cells following treatment with AuNPs (values indicate mean with SEM; n=3; lines represent linear quadratic curve fit).

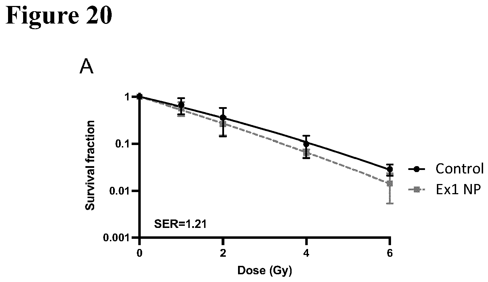

Figure 20: radiosensitising efficacy of functionalised nanoparticle formulation (corresponding to the formulation prepared in Example 1) in C42B prostate cancer cells at kV energies *in vitro.* Cells were treated with or without 100 µg/ml nanoparticles 24 hrs prior to irradiation (0-6 Gy). Clonogenic survival assays confirm significant radiosensitisation of C42B prostate cancer cells following treatment with nanoparticles (values indicate mean with SD; n=3; lines represent linear quadratic curve fit from which sensitiser enhancement ratios (SER) were determined, ratio of control to nanoparticle-treated).

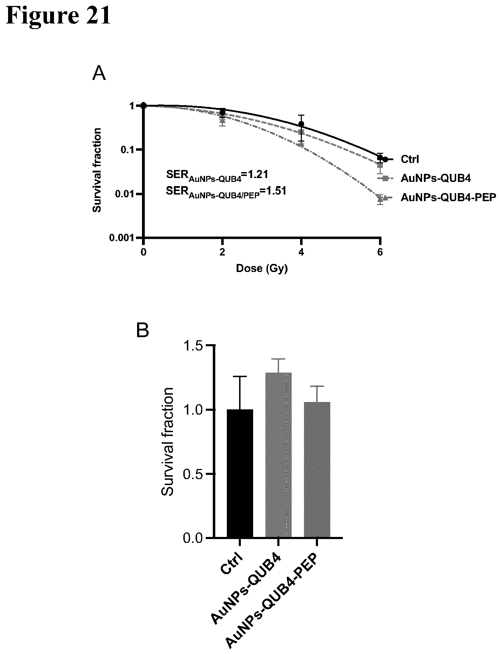

Figure 21: radiosensitising efficacy and cytotoxicity of functionalised nanoparticle formulations synthesised with alternative stabilising agent, pHPMA, in prostate cancer cells at kV energies *in vitro.* Cells were treated with or without 100 µg/ml of AuNP-QUB4-Pep 24 hrs prior to irradiation (0-6 Gy). Clonogenic survival assays confirm significant radiosensitisation of PC3 prostate cancer cells following treatment with AuNP-QUB4-Pep (values indicate mean with SD; n=3; lines represent linear quadratic curve fit from which sensitiser enhancement ratios (SER) were determined; ratio of control to nanoparticle-treated); (b) survival fractions of PC3 prostate cancer cells 14-days post treatment with 100 µg/ml of AuNP-QUB4-Pep formulations as determined by clonogenic survival assay (bars indicate mean with SD, n=3).

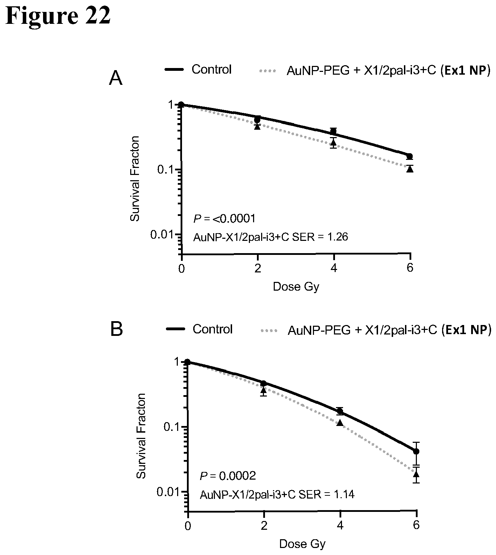

Figure 22: radiosensitising efficacy of functionalised nanoparticle formulation (corresponding to the formulation prepared in Example 1) in prostate cancer cells at MV energies *in vitro.* Cells were treated with or without 100 µg/ml nanoparticles 24 hrs prior to irradiation (0-6 Gy). Clonogenic survival assays confirm significant radiosensitisation of (a) PTEN-expressing DU145 and (b) PTEN-null PC3 prostate cancer cells following treatment with nanoparticles (values indicate mean with SD; n=3 (DU145, PC3); lines represent linear quadratic curve fit from which sensitiser enhancement ratios (SER) were determined, ratio of control to nanoparticle-treated; statistical significance determined by two-way ANOVA).

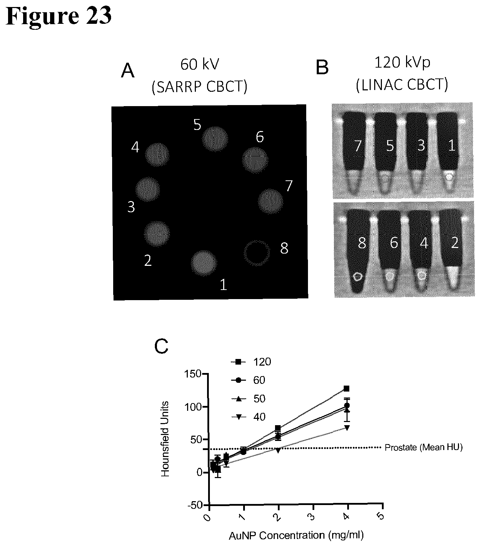

Figure 23: functionalised nanoparticle formulation (corresponding to the formulation prepared in Example 1) CT image potential: CBCT images for the nanoparticles at concentrations of 4 mg/ml (1), 2 mg/ml (2), 1 mg/ml (3), 0.5 mg/ml (4), 0.250 mg/ml (5), 0.125 mg/ml (6), water (7) and air (8) at (a) 60 kV and 10 kVp energies; (c) nanoparticle x-ray attenuation reported as Hounsfield Units (HU).

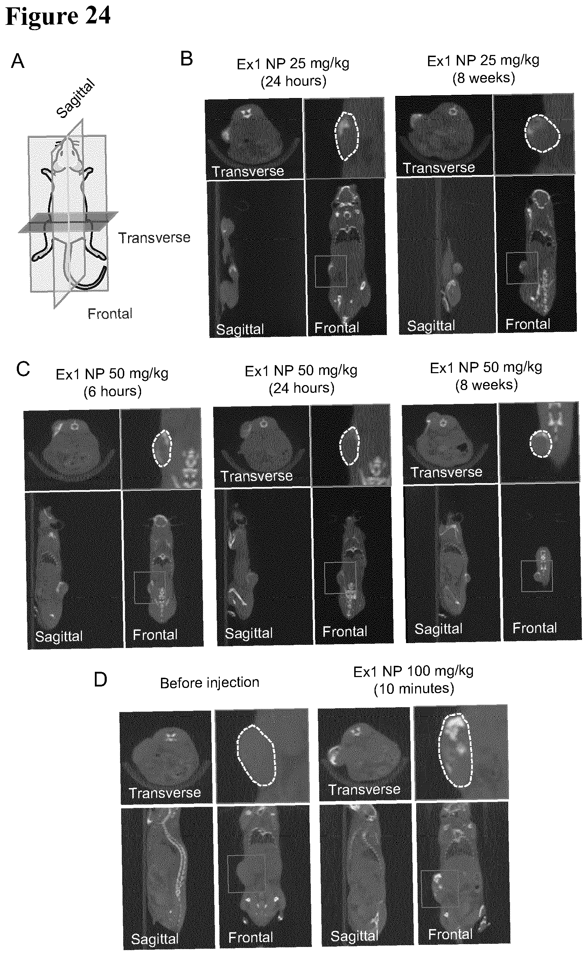

Figure 24: functionalised nanoparticle formulation (corresponding to the formulation prepared in Example 1) CT image potential *in vivo;* (a) schematic showing imaging planes in the sagittal, transverse and frontal; representative CBCT images (SARRP) at times indicated before and after intratumoral injection with (b) 25mg/kg, (c) 50mg/kg or (d) 100 mg/kg nanoparticle formulation, wherein the concentration of nanoparticles in the nanoparticle formulation was 30 g/L based on the amount of gold; top right image is an enlargement of the box on frontal image plane (all images obtained at 60 kV).

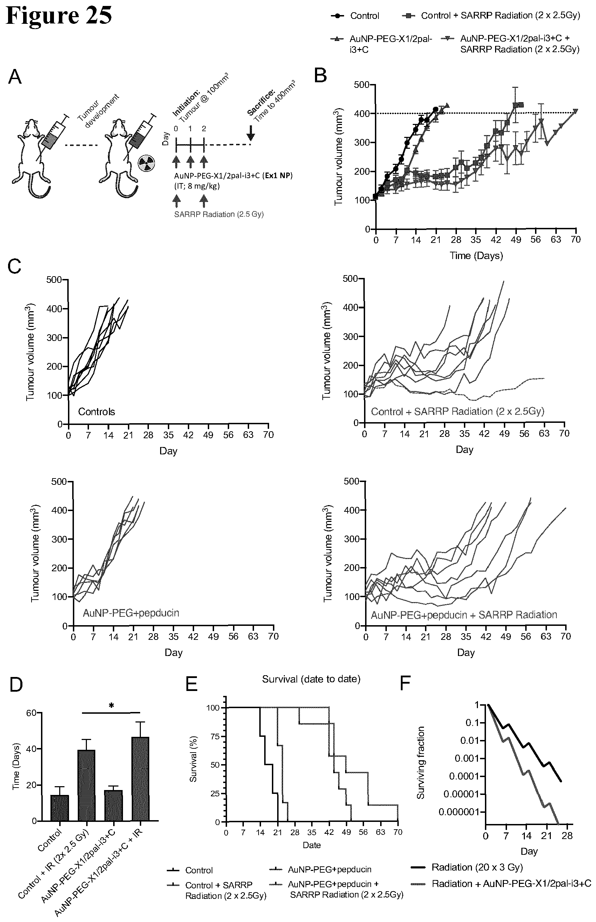

Figure 25: effect of functionalised nanoparticle formulation (corresponding to the formulation prepared in Example 1) alone or in combination with radiotherapy in PC3 prostate tumour bearing animals *in vivo:* (a) schematic depicting experimental design; 100 mm³ PC3 bearing SCID mice were injected intratumourally with nanoparticles (8 mg/kg) on days 0, 1 and 2 in combination with irradiation (IR) on days 0 and 2 (5 Gy total); (b) tumour growth curves showing tumour volume in PC3 bearing SCID mice (values shown for mean with SD; n=8 (control + IR), n=6 (nanoparticle), n=7 (nanoparticle + IR); statistical significance determined by one-way ANOVA with Sidak multiple comparison test); (c) individual growth curves for control, IR, nanoparticle and nanoparticle + IR treated animals; (d) time for tumours to triple in size following treatment (bars indicate mean with SD, from experiment in (b); statistical significance determined by one-way ANOVA with Tukey multiple comparison test); (e) Kaplan-Meier curve showing time to experimental end point (tumour volume >400 mm³) in each treatment group from (b); (f) computational modelling of tumour response to IR incorporating a 20% dose enhancement to simulated CHHiP trial hypofractionated treatment schedules (60 Gy, 20 fractions, 4 weeks).

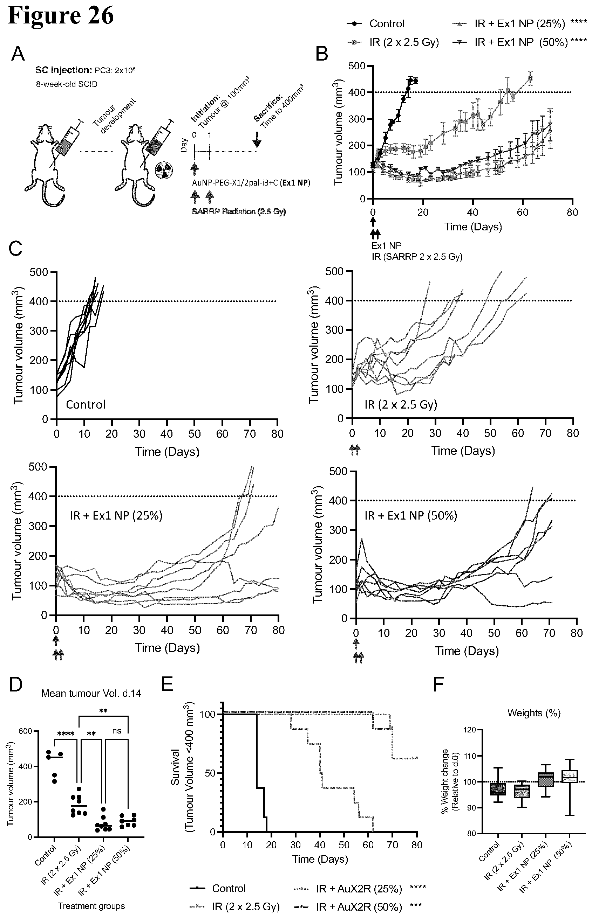

Figure 26: effect of functionalised nanoparticle formulation (corresponding to the formulation prepared in Example 1) alone or in combination with radiotherapy in PC3 prostate tumour bearing animals *in vivo:* (a) schematic depicting experimental design; when tumour volume reached 100 mm³ PC3 bearing SCID mice were injected intratumourally with nanoparticle dose volume at 25 or 50% tumour volume (equivalent to 25 or 50mg/kg respectively) on day 0 in combination with irradiation (IR) on days 0 and 1 (5Gy total); (b) tumour growth curves showing tumour volume in PC3 bearing SCID mice (values shown for mean with SD; n=8 (Control, IR, IR + nanoparticle 25%), n=7 (IR + nanoparticle 50%); statistical significance determined by one-way ANOVA with Sidak multiple comparison test); (c) individual growth curves for control, IR, IR + nanoparticle 25% and IR + nanoparticle 50% treated animals; (d) mean tumour volume at day 14 (points indicate individual animals, line represents mean, from experiment in (b); statistical significance determined by one-way ANOVA with Tukey multiple comparison test); (e) Kaplan-Meier curve showing time to experimental end point (tumour volume >400 mm³) in each treatment group from (b); (f) percentage change in animal weight, relative to day 0.

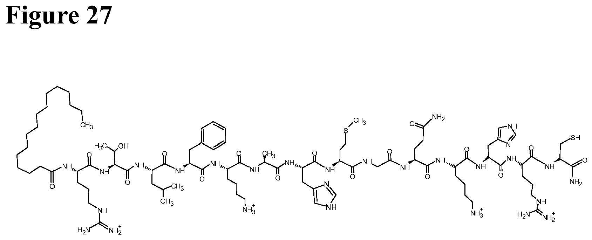

Figure 27: structure of pepducin of the invention.

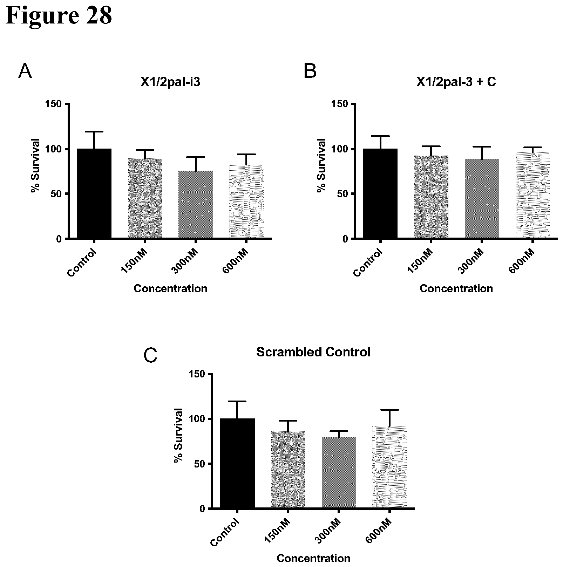

Figure 28: cell viability of PCa cell lines following exposure to pepducins.

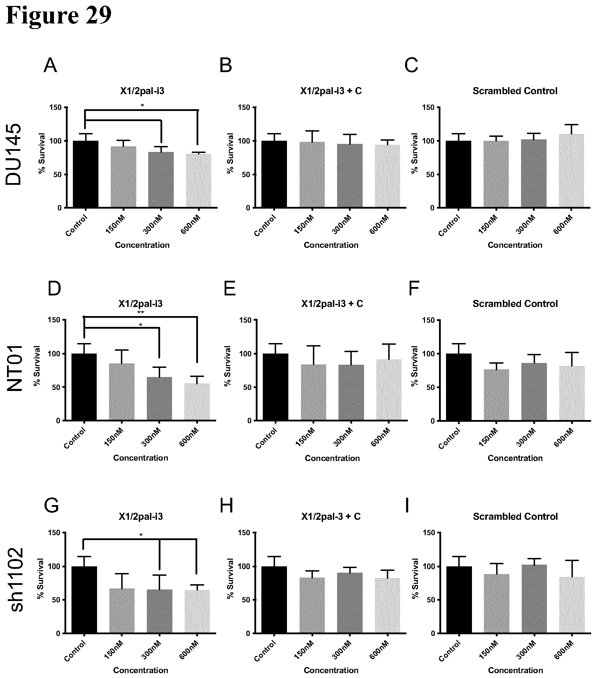

Figure 29: cell viability of DU145 cell lines following exposure to pepducins

### DETAILED DESCRIPTION

This application includes references to percent sequence identity. For the purpose of this invention, in order to determine the percent sequence identity of two sequences (such as two peptides or two amino acid sequences), the sequences are aligned for optimal comparison purposes (e.g. gaps can be introduced in a first sequence for optimal alignment with a second sequence). The nucleotide or amino acid residues at each position are then compared. When a position in the first sequence is occupied by the same nucleotide or amino acid as the corresponding position in the second sequence, then the nucleotides or amino acids are identical at that position. The percent sequence identity between the two sequences is a function of the number of identical positions shared by the sequences (i.e. % sequence identity = number of identical positions /total number of positions in the reference sequence × 100). Typically, the sequence comparison is carried out over the length of the reference sequence. For example, if the user wished to determine whether a given ("test") sequence is 95% identical to SEQ ID 1, SEQ ID 1 would be the reference sequence. To assess whether a sequence is at least 95% identical to SEQ ID 1 (an example of a reference sequence), the skilled person would carry out an alignment over the length of SEQ ID 1, and identify how many positions in the test sequence were identical to those of SEQ ID 1. If at least 95% of the positions are identical, the test sequence is at least 95% identical to SEQ ID 1. If the sequence is shorter than SEQ ID 1, the gaps or missing positions should be considered to be non-identical positions. The skilled person is aware of different computer programmes that are available to determine the homology or identity between two sequences. For instance, a comparison of sequences and determination of percent sequence identity between two sequences can be accomplished using a mathematical algorithm. Thus, the percent sequence identity between two amino acid or nucleic acid sequences may be determined using the Needleman and Wunsch (1970) algorithm which has been incorporated into the GAP programme in the Accelrys GCG software package (available at http://www.accelrys.com/products/gcg/), using either a Blosum 62 matrix or a PAM250 matrix, and a gap weight of 16, 14, 12, 10, 8, 6, or 4 and a length weight of 1, 2, 3, 4, 5, or 6.

### Nanoparticle

The present invention involves nanoparticles, which are generally solid particles having a size in the nano range, e.g. up to 100 nm, or more specifically from 1 to 100 nm.

Neither the size of the nanoparticle nor the size of the core is particularly limited. Thus, the core itself typically has a mean hydrodynamic diameter of from about 5 to about 50 nm when measured using dynamic light scattering in accordance with ISO 22412:2017. Preferably, the mean hydrodynamic diameter of the core is from about 10 to about 45 nm, more preferably about 15 to about 40 nm, even more preferably about 20 to about 30 nm. For instance, the core may have a mean hydrodynamic diameter of from about 22 to about 30 nm, such as about 26 nm.

The nanoparticle is preferably for use in treating cancer. The type of cancer is not particularly limited, but the treatment of prostate, breast and lung cancer are preferred. The treatment of prostate cancer is particularly preferred.

The nanoparticle is preferably a metal nanoparticle, such as a high atomic number metal nanoparticle. Thus, typically, the core comprises a metal.

The functionalised nanoparticle - including the core and functional agents - typically has a hydrodynamic dimeter of from about 20 to about 100 nm when measured using dynamic light scattering in accordance with ISO 22412:2017. Preferably, the mean hydrodynamic diameter of the core is from about 20 to about 80 nm, more preferably about 30 to about 60 nm, even more preferably about 40 to about 50 nm. For instance, the core may have a mean hydrodynamic diameter of from about 40 to 53 nm, such as 46 nm.

The standard deviation of the mean hydrodynamic diameter when measured using dynamic light scattering in accordance with ISO 22412:2017 is typically from about ±1 to about ±10 nm. For instance, if the core has a mean hydrodynamic diameter of about 26 nm, the standard deviation may be about ±3 nm, such as ±3.3 nm. Similarly, if the nanoparticle has a mean hydrodynamic diameter of about 46 nm, the standard deviation may be about ±6 nm, such as ±6.6 nm.

The nanoparticle of the invention is preferably a radiosensitising nanoparticle particularly useful for anticancer therapy as it has been shown to increase the tumour cell killing effect of radiotherapy. The significance of this is that the radiation dose required to achieve an equivalent therapeutic response is reduced, thus reduced the harmful side-effects to the patient being treated. Hence, in addition to providing a new and efficacious treatment for cancer, reduced negative also impact to the patient, such as side-effects caused by radiation, can be reduced.

The nanoparticle of the invention comprises a core that is functionalised with at least two different types of surface modification. The core is typically solid. Typically, the core is spherical or approximately spherical in shape. The surface modifications, or functional agents, are:
(i) a stabilising agent that can typically (a) improve biocompatibility, and/or (b) reduce opsonisation by proteins, and/or (c) impair AuNP uptake by macrophages, and/or (d) limit liver accumulation and toxicity; and
(ii) a targeting agent that can typically (a) antagonises the target receptors, such as CXCR1/2, to switch off pro-survival and/or pro-tumour signalling, and/or (b) simultaneously promotes nanoparticle internalisation.

The nanoparticle of the present invention is typically a high atomic number nanoparticle, i.e. typically the core comprises a chemical element having a high atomic number.

Through pre-clinical testing and recent clinical studies, it has been shown that high atomic number nanoparticles offer a means to enhance imaging, more accurately defining tumour boundaries, thus facilitating the precision of radiotherapy. Additionally, through superior mass absorption, high atomic number nanoparticles hold the potential to enhance the radiosensitivity of tumour cells, generating highly localised low-energy electrons through the physical interaction of ionising radiation with nanoparticles. Hence, the invention is typically associated with these benefits.

The various applications of the nanoparticles of the invention in biology are rooted in their unique physicochemical properties, and are not limited to size, shape, surface plasmon resonance (SPR) or surface chemistry of the nanoparticles. The nanoparticles of the invention are highly biocompatible, making them perfect candidates for use in disease diagnostics and treatment, either as delivery or contrast agents, or therapeutic sensitisers in their own right.

The nanoparticles of the invention may be radiosensitisers. As radiosensitisers, the nanoparticles confer their effects through the combination of two primary processes: physical and chemical radiosensitisation. Principally, high atomic number chemical elements such as gold and platinum efficiently absorb ionising radiation, destabilising the inner shell electrons of the chemical element and causing an electron to be ejected in the form of a photoelectron. In this state, the chemical element atom is inherently unstable and as such, electrons from outer shells move into inner shell orbitals, restabilising the atom. In the process of doing so, low-energy Auger electrons are liberated causing either direct damage (physical radiosensitisation), or chemically interacting with water to produce damaging hydroxy radicles, which are a product of radiolysis (chemical radiosensitisation). These are discussed in more detail above.

The low-energy Auger electrons and free radicals possess a range of 10's of nanometres, thus increasing damage to intracellular structures on a highly localised scale. It is this combination of physical and chemical interactions between the nanoparticle and the radiation source that predominately confers the radiation dose enhancing effects.

The nanoparticles of the invention are preferably biocompatible. Thus, biocompatibility is another feature that makes the invention particularly applicable as a radiosensitiser. The primary targeting which limits damage to surrounding normal tissue is the fact that radiotherapy itself is a highly targeted treatment modality, with complex radiotherapy treatment plans designed to minimise dose to surrounding normal tissue. This coupled with the fact that the nanoparticles of the invention typically confer little to no direct toxicity, means that if the nanoparticles of the invention are not contained within the tumour, the risk of any adverse off-target effect will typically be minimised.

Preferably, the nanoparticle comprises a core that comprises elemental gold, a stabilising agent that comprises a thiol-terminated polyethylene glycol, and a targeting agent that comprises a pepducin having an N-terminal lipid moiety derived from a C10-C25 fatty acid, a C-terminal cysteine residue and a peptide chain that has at least about 80% sequence identity to SEQ ID 1. Typically, the polyethylene glycol has a weight average molecular weight of about 4000 to about 6000 g/mol, the pepducin peptide chain has at least about 90% to 95% sequence identity to SEQ ID 1, and the C10-C25 fatty acid is selected from palmitic acid, myristic acid, stearic acid and lithocholic acid. Typically, the nanoparticle in this regard is obtainable by combining the elemental gold core with:
(i) the thiol-terminated polyethylene glycol and the pepducin, or
(ii) a thiol-terminated polyethylene glycol precursor and the pepducin, or
(iii) the thiol-terminated polyethylene glycol and a pepducin precursor, or
(iv) a thiol-terminated polyethylene glycol precursor and a pepducin precursor,
wherein the thiol-terminated polyethylene glycol, or precursor thereof, and the pepducin, or precursor thereof, are added respectively at a molar ratio of from about 1:2 to about 1:5, preferably about 1:3.

For instance, the nanoparticle may consist of an elemental gold core that is functionalised with a thiol-terminated polyethylene glycol and a pepducin having an N-terminal lipid moiety derived from a C10-C25 fatty acid, a C-terminal cysteine residue and a peptide chain that has at least about 90% sequence identity to SEQ ID 1, such as a pepducin corresponding to SEQ ID 2 or SEQ ID 3. Typically, the polyethylene glycol has a weight average molecular weight of about 4000 to about 6000 g/mol, the pepducin peptide chain has at least about 95% sequence identity to SEQ ID 1, and the C10-C25 fatty acid is selected from palmitic acid, myristic acid, stearic acid and lithocholic acid. Typically, the molar ratio of the thiol-terminated polyethylene glycol and pepducin (or precursors thereof) added to obtain the functionalised nanoparticle is about 1:2 to about 1:5, such as about 1:3.

### Stability

The functionalised nanoparticles of the invention typically enjoy excellent stability, particularly under extreme / biological conditions. This makes them particularly viable as options for medical treatments, especially when considered in combination with the typically excellent cellular uptake thereof.

Thus, the nanoparticle of the invention is preferably stable within physiological conditions. Typically, the nanoparticle is stable for at least 2 months. Preferably, the nanoparticle enjoys improved stability as compared against the corresponding nanoparticle lacking either the stabilising agent or lacking both the targeting agent and the stabilising agent.

### Cellular uptake

The functionalised nanoparticles of the invention typically enjoy excellent cellular uptake / internalisation. They typically also enjoy excellent target specificity. This makes them particularly viable as options for medical treatments, especially when considered in combination with the typically excellent stability thereof.

Thus, the nanoparticle of the invention is preferably one that enjoys improved cellular uptake as compared against the corresponding nanoparticle lacking either the targeting agent or lacking both the targeting agent and the stabilising agent.

### Nanoparticle core

The core is the central component of the nanoparticle. In other words, the main body or nucleus of the nanoparticle. The core has an outer surface which may be functionalised with one or more agents. The terms "core" and "nanoparticle core" may be used interchangeably. The core typically has a mean hydrodynamic diameter as measured using dynamic light scattering of about 5 to about 50 nm.

The nanoparticles of the invention comprise a core that is functionalised with a stabilising agent and a targeting agent. The nanoparticles are preferably high atomic number nanoparticles, i.e. the nanoparticle core typically comprises a high atomic number chemical element. A high atomic number chemical element is an element having a higher atomic number than iodine. Typically, this is a chemical element having an atomic number of from about 44 to about 83. For instance, the high atomic number chemical element may be one having an atomic number of from about 54 to about 83. Preferably, the high atomic number chemical element is one having an atomic number of from about 64 to about 83. It is noted that references to chemical elements include isotopes thereof and encompass such elements in elemental form, including allotropes thereof, and in non-elemental form.

Typically, the atomic density of the high atomic number chemical element is greater than about 4.5 g cm⁻³. Typically, the atomic density of the high atomic number chemical element is less than about 50 g cm⁻³. For instance, the atomic density of the high atomic number chemical element may be between about 4.5 to 30 g cm⁻³, preferably between about 5 to 25 g cm⁻³, more preferably about 8 to 20 g cm⁻³.

The chemical element is typically present in the core in either elemental form (i.e. as the elementary substance) or non-elemental form (e.g. as part of a compound containing one or more different elements, such as an oxide, nitride, nitrate, sulphide, sulphate, etc.).

Preferably, the high atomic number chemical element is a noble metal. For instance, the high atomic number chemical element may be a noble metal having an atomic number of from about 44 to about 83.

Exemplary high atomic number chemical elements include gold, platinum, iridium, osmium, bismuth, tantalum, thulium, hafnium and gadolinium. For instance, the core may comprise gold. The chemical element is preferably present in the core in elemental form when the chemical element is gold, platinum, iridium or osmium. The chemical element is preferably present in the core in non-elemental form when the chemical element is bismuth, tantalum, thulium, hafnium, or gadolinium. For bismuth, tantalum, thulium, hafnium, and gadolinium, oxides thereof are particularly preferred. Typical oxides in this regard include Gd₂O₃, Tm₂O₃, HfO₂, Ta₂O₅ and Bi₂O₃.

Typically, the core comprises elemental gold.

The core may consist of a single chemical element (or compound thereof), for example gold, platinum, iridium, osmium, a compound of bismuth (such as an oxide thereof), a compound of tantalum (such as an oxide thereof), a compound of thulium (such as an oxide thereof), a compound of hafnium (such as an oxide thereof), or a compound of gadolinium (such as an oxide thereof). The core may consist of gold, platinum, iridium, or osmium. The core may consist of elemental gold.

The core may consist of more than one chemical element. For example, the core may comprise one or more of: gold, platinum, iridium, osmium, a compound of bismuth (such as an oxide thereof), a compound of tantalum (such as an oxide thereof), a compound of thulium (such as an oxide thereof), a compound of hafnium (such as an oxide thereof), and/or a compound of gadolinium (such as an oxide thereof).

Where the core comprises a high atomic number chemical element, which is typical, it may also comprise another chemical element, such as a metal. For instance, the core may comprise a first section comprising a metallic material, such as iron, and a second section comprising a high atomic number chemical element, such as gold. For instance, the first section may be located radially inwards of the second section, such that the first section may be considered as the nucleus of the core, with the second section being the membrane / coating / outer layer thereof.

Thus, the core may comprise a magnetic component that imbues magnetic properties to the nanoparticle. This is typically achieved by making the core from a magnetic chemical element in addition to the high atomic number chemical element. Exemplary magnetic chemical elements in this regard include iron, nickel and cobalt. For instance, this can be achieved by using a core that has an iron "nucleus" that is coated with a gold outer layer. Imbuing the nanoparticles of the invention with magnetic properties can aid their systemic delivery / targeting.

Typically, the high atomic number chemical element is one other than silver or mercury. Often, the core does not comprise silver. Often, the core does not comprise mercury.

Typically, the core is a solid core. The shape of the core is not particularly limited. For instance, non-limiting examples of possible core shapes include spherical, approximately spherical, rod-shaped, approximately rod-shaped, star-shaped, approximately star-shaped, pyramid-shaped, and approximately pyramid shaped. Typically, the core is spherical or approximately spherical.

The nanoparticle core has an outer surface. It is to this surface that the stabilising agent and the targeting agent are typically attached.

### Nanoparticle core functionalisation

The core has two or more functional agents that are bio-conjugated, connected, coordinate covalently (datively) bonded, covalently bonded, ionically bonded, crosslinked, attached, and/or otherwise linked thereto. Typically, the functional agents are surface modifications of the nanoparticle core. These functional agents include the stabilising and targeting agents of the invention.

The stabilising agent is one functional agent attached to the core, preferably to the outer surface of the core, that forms part of the functionalised nanoparticle of the invention. The stabilising agent typically enhances the stability of the nanoparticles, including by preventing agglomeration, deterioration, and so on.

The targeting agent is another functional agent attached to the core, preferably to the outer surface of the core, that forms part of the functionalised nanoparticle of the invention. The targeting agent typically enables enhanced and specific cellular uptake of nanoparticles in cells expressing chemokine receptors, thus improving both the specificity and efficacy of nanoparticle-based treatments.

The nanoparticle core is functionalised with a stabilising agent and a targeting agent. Additional functional agents, such as imaging agents and cytotoxic agents, may also be present. If present, these are typically attached to the outer surface of the core.

Attachment of the stabilising agent (e.g. polyethylene glycol (PEG), chitosan, polyethylenimine (PEI), poly(N-(2-hydroxypropyl) methacrylamide (pHPMA)) typically ensures (a) improved biocompatibility, and/or (b) reduced opsonisation by proteins, and/or (c) impaired nanoparticle uptake by macrophages, and/or (d) limited liver accumulation and toxicity. Attachment of the targeting agent typically (a) antagonises the target receptors (typically the CXCR1/2 receptors) to switch off pro-survival and/or pro-tumour signalling, and/or (b) promotes nanoparticle internalisation / cellular uptake.

The molar ratio of the stabilising agent to the targeting agent is the molar ratio of the amount of stabilising agent that is added during nanoparticle synthesis relative to the amount of targeting agent that is added during nanoparticle synthesis, specifically when the nanoparticle is being functionalised. In other words, this is the molar ratio of stabilising agent to targeting agent used in step (b) of the nanoparticle preparation process of the invention. This term is used interchangeably to refer to the molar ratio of the (i) stabilising agent to targeting agent, (ii) stabilising agent precursor to targeting agent, (iii) stabilising agent to targeting agent precursor, and (iv) stabilising agent precursor to targeting agent precursor. Typically, the term refers to molar ratio of the stabilising agent to targeting agent.

The molar ratio is typically from about 1:2 to about 1:5. Thus, the targeting agent (or precursor thereof) is usually combined with the core during the functionalisation step of the preparation process in an amount that is, on a molar basis, two to five times greater than the amount of stabilising agent that is added. The molar ratio (stabilising agent / precursor to targeting agent / precursor) may be from about 1:2 to about 1:4. A particularly preferred molar ratio is about 1:3.

The manner in which the stabilising and targeting agents are attached to the core is not particularly limited, and the two may be attached via the same or different bonding interactions. Typically, the stabilising and targeting agents are, independently, attached to the core via one or more of: dative (coordinate covalent) interactions, covalent interactions, electrostatic (ionic) interactions, and conjugation chemistry e.g. via crosslinkers.

Typically, the stabilising and targeting agents comprise specific moieties that enable the creation of self-assembled monolayers on the surface of the core (i.e. at the interface between the core and the stabilising and targeting agents). For instance, this can be achieved via thiol-termination of the stabilising and targeting agents when the core comprises gold. This is due to the formation of Au-S dative bonds between Au atoms in the core and S atoms in the thiol groups. Under certain pH conditions, the dative bonds can shift to true covalent bonds, thus providing further strength to the attachment.

Routine covalent bonding interactions are also possible, as are electrostatic interactions. For example, citrate reduced gold nanoparticles have an overall negative charge, usually around -9.4 mV. Therefore, any positively charged stabilising and targeting agents will electrostatically interact with the negative gold core enabling functionalisation. However, dissociation between the core and stabilising and targeting agents can occur relatively easily, especially at specific pH values (e.g. if the targeting agent is a protein, at pH values above the isoelectric point of said protein) and at high salt concentrations.

Crosslinker chemistry, such as amine-reactive crosslinker chemistry, can also be used to attach the stabilising and targeting agents to the nanoparticle core. Molecules such as EDC (1-ethyl-3-(3-dimethylaminopropyl)carbodiimide hydrochloride) can be utilised to enable amine and carboxyl coupling between the core and functional groups. If the targeting agent is a peptide, for instance, carboxyl nanoparticles can be conjugated to the amine moieties of the peptide.

Preferably, both the stabilising and targeting agents are attached to the core via dative bonds. When the core comprises gold and the stabilising and/or targeting agent is thiol-terminated, attachment is preferably via an Au-S dative bond.

The skilled person can readily select suitable interactions for attach specific stabilising and targeting agents to specific cores using means available to them in the art and their common general knowledge.

### Nanoparticle stabilising agent

The stabilising agent is added to the nanoparticles in order to stabilise them under extreme or biologically relevant conditions. Inclusion of the stabilising agent in the nanoparticle typically results in one or more of: improved biocompatibility, reduced opsonisation by proteins, and/or impaired uptake by macrophages preventing the formation of protein-coronas.

The stabilising agent may comprise a natural polymer, synthetic polymer, biocompatible polymer, biomedical polymer, hydrophilic polymer, or a combination thereof. Optionally, the stabilising agent comprises a thiol-terminated species. For instance, if the stabilising agent comprises a natural polymer, synthetic polymer, biocompatible polymer, biomedical polymer, and/or hydrophilic polymer, then said natural polymer, synthetic polymer, biocompatible polymer, biomedical polymer, and/or hydrophilic polymer may be thiol-terminated. Typically, the stabilising agent comprises a thiol-terminated species.

Typically, the stabilising agent comprises polyethylene glycol, polyethyleneimine, poly(N-(2-hydroxypropyl) methacrylamide, chitosan, or a combination thereof. The polyethylene glycol, polyethyleneimine, poly(N-(2-hydroxypropyl) methacrylamide, and/or chitosan are preferably thiol-terminated in this regard.

Preferably, the stabilising agent comprises polyethylene glycol. Particularly preferably, the stabilising agent is polyethylene glycol. The polyethylene glycol may optionally be thiol-terminated. Preferably, the polyethylene glycol is thiol-terminated.

In this regard, the polyethylene glycol typically has a weight average molecular weight (M_{w}) of from about 800 to about 10000 g/mol. Exemplary polyethylene glycol weight average molecular weights include 800 g/mol, 2000 g/mol and 5000 g/mol. Preferably, the polyethylene glycol typically has a weight average molecular weight of from about 1000 to about 7000 g/mol, more preferably from about 2000 to about 6000 g/mol. Particularly preferably, the polyethylene glycol has a weight average molecular weight of around 5000 g/mol.

### Nanoparticle targeting agent

The targeting agent is added to the nanoparticles in order to preferably enhance target specificity, and/or uptake to / penetration of desired cells, and/or blocking of the activation of specific signalling pathways.

Preferably, the targeting agent is not RME peptide (CKKKKKKSEDEYPYVPN) or H5WYG peptide (GCGLFHAIAHFIHGGWHGHHGWYG). Preferably, the targeting agent is a cell-penetrating agent.

Optionally, the targeting agent comprises an arginine residue. Optionally, the targeting agent comprises a peptide chain having between 3 and 15 amino acid residues or a peptide chain having between 20 to 50 amino acid residues.

The targeting agent may comprise a peptide and/or small molecule. The peptide is typically a pepducin, protein, polypeptide, oligopeptide, aptamer, antibody, and/or antibody fragment, and preferably is a pepducin or a protein. The small molecule is typically a small molecule inhibitor, such as a small molecule inhibitor that targets a chemokine receptor, e.g. CXCR1 or CXCR2. Exemplary such small molecules that target CXCR1 or CXCR2 include AZD5069 (CAS No.: 878385-84-3), SCH-527123 (Navarixin, MK-7123, CAS No.: 473727-83-2), SCH-479833, L (CAS No.: 473725-29-0), adarixin (DF2156A, CAS No.: 849776-05-2), and Reparixin (CAS No.: 266359-83-5).

Typically, the targeting agent comprises a peptide. For instance, the targeting agent may be, or comprise, a peptide. Preferably, the targeting agent comprises or is a pepducin.

When the targeting agent comprises or is a pepducin, said pepducin typically comprises a peptide chain having fewer than about 50 amino acid residues and an N-terminal lipid moiety, wherein the the peptide chain has a C-terminal cysteine residue. Preferably, the pepducin does comprise a peptide chain having fewer than about 50 amino acid residues, an N-terminal lipid moiety and a C-terminal cysteine residue. Typically, the N-terminal lipid moiety is a moiety derived from a fatty acid, preferably a C10-C25 fatty acid.

The pepducin peptide chain typically has at least about 3 amino acid residues, preferably at least about 5 amino acid residues, more preferably at least about 10 amino acid residues. The peptide chain typically has fewer than about 50 amino acid residues, preferably fewer than about 40 amino acid residues, more preferably fewer than about 30 amino acid residues. For instance, the pepducin peptide chain may have about 3 to about 50 amino acid residues, preferably about 5 to about 40 amino acid residues, more preferably about 10 to about 30 amino acid residues, such as around 11 to 20 amino acid residues.

The pepducin peptide chain is typically derived from the amino acid sequence of an intracellular loop domain of a G protein coupled receptor, such as a chemokine receptor. Exemplary chemokine receptors in this regard include CXCR1 and CXCR2.

Typically, the pepducin peptide chain comprises an amino acid sequence having at least about 80% sequence identity to SEQ ID 1. Preferably, the pepducin peptide chain comprises an amino acid sequence having at least about 85% sequence identity to SEQ ID 1, more preferably at least about 90% sequence identity, even more preferably at least about 95% sequence identity. For instance, the pepducin peptide chain may comprise an amino acid sequence having about 99% sequence identity to SEQ ID 1. Particularly preferably, the pepducin comprises a peptide chain that corresponds to SEQ ID 1.

Preferably, the pepducin peptide chain consists of an amino acid sequence having at least about 80% sequence identity to SEQ ID 1, such as an amino acid sequence having at least about 85% sequence identity, preferably at least about 90% sequence identity, even more preferably at least about 95% sequence identity to SEQ ID 1. For instance, the pepducin peptide chain may consist of an amino acid sequence having about 99% sequence identity to SEQ ID 1. Particularly preferably, the entire peptide chain element of the pepducin corresponds to SEQ ID 1.

The N-terminal lipid moiety of the pepducin is typically a C10-25 lipid moiety, i.e. a lipid moiety containing from 10 to 25 carbon atoms in total. For instance, the lipid moiety may be a C12-20 lipid moiety, such as a C14 to 18 lipid moiety. Exemplary chain lengths include C14, C16 and C24 lipid moieties. Typically, the lipid moiety is derived from a fatty acid. Exemplary fatty acids in this regard include palmitic acid, myristic acid, stearic acid and lithocholic acid. Exemplary derivatives in this regard include carbonyl forms of the fatty acids (such that they are bonded to the peptide chain via an amide linkage between the carbonyl of the fatty acid and a nitrogen of the peptide chain) and carboxylate forms of the fatty acids, and so on. Typically, the derivative is a carbonyl form of the fatty acid.

Preferably, the lipid moiety is derived from palmitic acid. More preferably, it is a palmitoyl moiety. The N-terminal lipid moiety of the pepducin may be attached to the peptide chain of the pepducin using any conventional means known in the art.

The C-terminal cysteine residue of the pepducin peptide chain is typically amidated. Preferably, it is amidated.

Thus, the targeting agent typically comprises a pepducin having fewer than about 50 amino acid residues, an N-terminal lipid moiety derived from palmitic acid, myristic acid, stearic acid and lithocholic acid, and a C-terminal cysteine residue, wherein the pepducin comprises a peptide chain having at least about 80% sequence identity to SEQ ID 1, preferably at least about 90% sequence identity, more preferably at least about 95% sequence identity. Preferably, the targeting agent is a pepducin having fewer than about 50 amino acid residues, an N-terminal lipid moiety derived from palmitic acid, myristic acid, stearic acid and lithocholic acid, and a C-terminal cysteine residue, wherein the pepducin comprises a peptide chain having at least about 80% sequence identity to SEQ ID 1, more preferably at least about 90% sequence identity, more preferably at least about 95% sequence identity. For instance, the targeting agent may be a pepducin having an N-terminal lipid moiety derived from palmitic acid, such as a palmitoyl moiety, and a peptide chain having at least about 80% sequence identity to SEQ ID 1, preferably at least about 90% sequence identity, more preferably at least about 95% sequence identity. Thus, as is often preferred, the targeting agent may be a pepducin corresponding to SEQ ID 2, or a pepducin corresponding to SEQ ID 3, or a pepducin corresponding to both SEQ ID 2 / SEQ ID 3 (i.e. when the two are the same - in this regard, as will be readily understood by the skilled person, the C-terminus of the SEQ ID 2 amino acid chain may be amidated).

Optionally, the chemical structure of the targeting agent as attached to the nanoparticle core may correspond, or correspond substantially, to the targeting agent prior to addition to the nanoparticle core. For instance, the targeting agent may be the same in terms of its chemical structure both before and after attachment to the nanoparticle core. Thus, the targeting agent may comprise a pepducin having fewer than about 50 amino acid residues, an N-terminal lipid moiety derived from palmitic acid, myristic acid, stearic acid or lithocholic acid, and a C-terminal cysteine residue, wherein the peptide chain has at least about 80% sequence identity to SEQ ID 1, preferably at least about 90% sequence identity, more preferably at least about 95% sequence identity. Preferably in this regard, the lipid moiety is a palmitoyl moiety. For example, the targeting agent may be a pepducin having fewer than about 50 amino acid residues, an N-terminal lipid moiety derived from palmitic acid, myristic acid, stearic acid or lithocholic acid, and a C-terminal cysteine residue, wherein the peptide chain has at least about 80% sequence identity to SEQ ID 1, more preferably at least about 90% sequence identity, more preferably at least about 95% sequence identity. For instance, the targeting agent may be a pepducin corresponding to SEQ ID 2 and/or SEQ ID 3.

### Pepducin of the invention

Pepducins (peptidomimetics, lipopeptides) are cell penetrating lipidated peptides that act as allosteric modulators. They were initially discovered by Kuliopus & Covic in the late 1990s. The peptide components of these pepducins (typically 10-20 amino acids in length) are based on the intracellular loops of GPCRs coupled to a hydrophobic moiety by a linker. Pepducins act to uncouple receptors from activating intrinsic G proteins: the cytoplasmic loops of GPCRs are critical sites of interaction with components of the intracellular signalling cascade. Pepducins can stabilise GPCRs in a particular conformational state thereby (i) affecting the ability of the receptor to undergo further conformational changes and (ii) affecting the ability of the receptor to interact with cellular signalling machinery, subsequently blocking activation of the signalling pathway.

In addition to a nanoparticle, the present invention relates to a novel pepducin. In particular, the present inventors have discovered a novel pepducin comprising a peptide chain and an N-terminal lipid moiety derived from a C10-25 fatty acid, wherein the peptide chain has at least about 90% sequence identity to SEQ ID 1 and wherein the peptide chain has a C-terminal cysteine residue, and wherein the pepducin comprises fewer than about 50 amino acid residues. Preferably, the peptide chain also has an N-terminal arginine residue.

Typically, the pepducin comprises a peptide chain having at least about 95% sequence identity to SEQ ID 1, preferably at least about 97% sequence identity, more preferably at least about 98% sequence identity. For instance, the pepducin may comprise a peptide chain having about 99% sequence identity to SEQ ID 1. Particularly preferably, the pepducin comprises a peptide chain corresponding to SEQ ID 1. Typically, the peptide component of the pepducin consists of a peptide chain corresponding to SEQ ID 1.

Typically, the lipid moiety is derived from palmitic acid, myristic acid, stearic acid or lithocholic acid. Typically, the lipid moiety is a carbonyl form of palmitic acid, myristic acid, stearic acid or lithocholic acid (such that it is bonded to the peptide chain via an amide linkage between the carbonyl of the fatty acid and a nitrogen of the peptide chain). Preferably, the lipid moiety is derived from palmitic acid. More preferably, the lipid moiety is a palmitoyl moiety.

Particularly preferably, the pepducin comprises a peptide chain having an N-terminal arginine residue and a C-terminal cysteine residue and having at least about 90% sequence identity to SEQ ID 1. Preferably, the pepducin peptide chain has at least about 95% sequence identity to SEQ ID 1, more preferably at least about 97% sequence identity, more preferably at least about 98% sequence identity. For instance, the peptide chain may have about 99% sequence identity to SEQ ID 1. Particularly preferably, the peptide chain corresponds to SEQ ID 1.

Thus, preferably, the pepducin comprises an N-terminal lipid moiety derived from palmitic acid, myristic acid, stearic acid or lithocholic acid and a peptide chain having an N-terminal arginine residue and a C-terminal cysteine residue and having at least about 90% sequence identity to SEQ ID 1. More preferably, the pepducin comprises an N-terminal lipid moiety that is a carbonyl form of palmitic acid, myristic acid, stearic acid or lithocholic acid and a peptide chain having an N-terminal arginine residue and a C-terminal cysteine residue and having at least about 90% sequence identity to SEQ ID 1.

For instance, the pepducin may comprise, or consist of, an N-terminal lipid moiety that is a derivative of palmitic acid and a peptide chain having an N-terminal arginine residue and a C-terminal cysteine residue, wherein the peptide chain has at least about 95% sequence identity to SEQ ID 1.

Preferably, the pepducin of the invention comprises an N-terminal palmitoyl moiety and a peptide chain having an N-terminal arginine residue and a C-terminal cysteine residue, wherein the peptide chain has at least about 95% sequence identity to SEQ ID 1 and no more than about 50 amino acid residues.

For instance, the pepducin of the invention may consist of an N-terminal palmitoyl moiety and a peptide chain having an N-terminal arginine residue and a C-terminal cysteine residue, wherein the peptide chain has at least about 95% sequence identity to SEQ ID 1 and no more than about 50 amino acid residues.

Particularly preferably, the pepducin corresponds to SEQ ID 3.

The structure of the pepducin of the invention when it corresponds to SEQ ID 3, which is particularly preferred, is depicted in Figure 27. This pepducin may be referred to as X1/2pal-i3+C (for instance in some of the Figures). The corresponding pepducin lacking the C-terminal cysteine residue (comparative example) may be referred to as X1/2pal-i3 (for instance in some of the Figures).

The pepducin of the invention targets the CXCR1/2 signalling pathway and exploits the tumour stress response to radiotherapy to improve the internalisation of AuX2R into the tumour. A typical EBRT radiotherapy treatment plan delivers 60Gy in twenty individual 3Gy fractions. Doses in this range have been proven to enhance the tumour expression of CXCR1/2, therefore radiotherapy will act to prime the tumour to overexpress the targeting receptor for any nanoparticle comprising the pepducin of the invention.

The pepducin of the invention is an antagonist of CXCR1/CXCR2. It is derived from the i3 intracellular loops of CXCR1/CXCR2, and has been shown to block CXCR2 signal transduction in *in vitro* and *in vivo* models of cancer.

The presence of the C-terminal cysteine residue enables conjugation of the pepducin to the nanoparticle core, which typically comprises gold. This in turn leads to a functionalised nanoparticle that can effectively target the CXCR1/2 signalling pathway, thus resulting in good target specificity and cellular uptake. Without the inclusion of the C-terminal cysteine residue, a less stable and functional nanoparticle is produced. The surprising technical benefits of the invention, i.e. efficient internalisation, potent *in vitro* and *in vivo* radiosensitisation, are therefore much harder to achieve.

Furthermore, the addition of the cysteine residue, does not negatively impact the existing functionality of known pepducins. More specifically, the pepducin of the present invention is as good a radiosensitiser, i.e. there is no statistical difference, as the corresponding pepducin lacking the C-terminal cysteine residue, which is known. The pepducin of the present invention additionally retains the molecular impact of supressing the anti-apoptotic protein Bcl-2. Surprisingly, it appears that the pepducin of the present invention has significantly less direct toxicity in DU145 cells than the corresponding pepducin lacking the C-terminal cysteine residue (see Example 9). This is a highly desirable property for a radiosensitiser, as the majority of the effect should only be observed in the presence of radiation.

The pepducin of the invention can be prepared by routine means. For instance, the pepducin of the invention may be prepared by functionalising a peptide chain having at least about 90% sequence identity to SEQ ID 1 (or the corresponding percentage sequence identity to obtain the relevant resultant pepducin) with a fatty acid or derivative thereof.

By way of example, when the lipid component is derived from palmitic acid, the pepducin may be prepared by palmitoylating a peptide chain having at least about 90% sequence identity to SEQ ID 1 with palmitic acid, leading to attachment of the palmitoyl moiety to the arginine residue at the N-terminus of the peptide chain.

### Compositions of the invention

The present invention is also directed to a composition comprising one or more nanoparticle(s) of the invention. The nature of said composition is not particularly limited.

For instance, it could be a standard composition, a pharmaceutical composition, a diagnostic composition, an experimental composition, and so on.

The nanoparticle concentration in the compositions of the invention is not particularly limited. Referring specifically to the amount (mass) of nanoparticle core in the composition, the concentration is typically about 20 µg/L to about 200 g/L, i.e. about 20 µg core per litre composition to about 200 g core per litre composition. By way of example, when the core is (i.e. consists of) gold, then this would correspond to (about) 20 µg to 200 g gold per litre composition.

Thus, the nanoparticle concentration may be about 100 µg/L to about 150 g/L, preferably about 500 µg/L to about 100 g/L, more preferably about 1 mg/L to about 60 g/L. For instance, the nanoparticle concentration may be about 1 mg/L to about 200 mg/L, such as about 50 mg/L, especially if the composition is to be used for *in vitro* research / experimental purposes. Alternatively, the nanoparticle concentration may be about 1 g/L to about 60 g/L, such as about 30 g/L, especially if the composition is to be used for *in vivo* research / experimental purposes or is a pharmaceutical or diagnostic composition.

The nanoparticle concentration may alternatively be defined in terms of molarity. Again, referring specifically to the amount (moles) of nanoparticle core in the composition, the concentration is typically about 0.1 µM to about 1 M, i.e. about 0.1 µM core per litre composition to about 1 M core per litre composition. By way of example, when the core is gold, then this would correspond to (about) 0.1 µM to 1 M gold per litre composition.

Thus, the nanoparticle concentration may be about 1 µM to about 500 mM, preferably about 4 µM to about 250 mM. For instance, the nanoparticle concentration may be about 5 µM to about 1 mM, such as about 500 µM, especially if the composition is to be used for *in vitro* research / experimental purposes. Alternatively, the nanoparticle concentration may be about 100 mM to about 200 mM, such as 150 mM, especially if the composition is to be used for *in vivo* research / experimental purposes or is a pharmaceutical or diagnostic composition.

Typically, the composition is a colloid and comprises one or more nanoparticle(s) of the invention and ultrapure water. The composition may consist of, or consist essentially of, ultrapure water and one or more nanoparticle(s) of the invention. The composition may comprise very small amounts of acetonitrile (<0.0025 %v/v).

Ultrapure (or Type I) water is that which has undergone a high level of purification and is defined (ASTM International) as having <50 ppb of total organic carbons (TOC), <0.03 EU/ml endotoxins, and bacteria (<1 CFU/ml) with nucleases (RNase & DNase) and proteases at non-detectable levels. Pre-clinical, research grade formulations of the nanoparticles of the invention are typically made in purified water satisfying the European Pharmacopoeia (Ph. Eur.) monograph 0008. Clinical grade formulations containing the nanoparticles of the invention, such as those intended for parenteral delivery, are typically prepared in water for injection (WFI) conforming to Ph. Eur. 0169.

When the composition is a pharmaceutical composition, the composition additionally comprises one or more pharmaceutically acceptable excipient(s). The nature of the excipient(s) is not particularly limited, and the skilled person can readily select suitable excipient(s) based on their common general knowledge. The pharmaceutical composition also comprises the nanoparticle(s) in a therapeutically effective amount.

When the composition is a diagnostic composition, the composition typically corresponds to the pharmaceutical composition. For example, the pharmaceutical composition may optionally be used as a diagnostic composition, and *vice versa.*

The composition, including the pharmaceutical composition, can be administered to the patient / subject using any conventional means known in the art. The skilled person can readily determine an appropriate administration method using routine tests and their common general knowledge.

### Kit

The present invention is also directed to a kit comprising one or more nanoparticle(s) of the invention and/or a composition of the invention in addition to means for administering the nanoparticle(s) or composition to a subject.

Typically, the kit comprises a delivery system comprising a 1 ml graduated glass syringe and a 18/20G luer lock delivery needle. The syringe is typically preloaded with a composition of the invention having a nanoparticle concentration as defined above of about 1 to about 60 g/L, such as about 30 g/L.

### Preparation (synthesis) of nanoparticles

The process of preparing the nanoparticles of the invention is not particularly limited. For instance, the nanoparticles may be prepared using so-called "top-down" approaches such as milling or laser ablation, so-called "bottom-up" approaches such as chemical reduction methods, or other methods known to be suitable to the skilled person.

Preferably, though, the nanoparticles are prepared using the specific preparation process of the invention. Thus, the present invention is also directed to a process of preparing a nanoparticle.

In particular, the nanoparticles are preferably prepared by the reduction of one or more metal ion(s) from their ionic salt(s) using one or more reducing agent(s). This synthetic approach is particularly advantageous given its tunability (based on reducing agent, stabilising agent, temperature and component ratio) and homogeneity.

The inventive process typically comprises the following steps:
(a) in a first step, combining a metal salt comprising a chemical element having an atomic number of from about 44 to about 83 with a reducing agent to obtain a dispersion comprising an unfunctionalised nanoparticle; and
(b) in a second step, adding to the dispersion obtained in step (a) a stabilising agent or precursor thereof and a targeting agent or precursor thereof at a molar ratio of from about 1:2 to about 1:5 respectively to obtain a functionalised nanoparticle.

In this regard, the chemical element, stabilising agent, targeting agent and molar ratios are all as defined above, including the typical and preferred features thereof.

This process can be used to prepare the nanoparticle of the present invention.

The process optionally comprises an additional step, step (d), wherein the functionalised nanoparticle obtained in step (b) - or, if applicable, step (c) (see discussion of step (c) below) - is dispersed in water, preferably ultrapure water, or an aqueous solution or a buffer to produce a colloid solution. In doing so, the process can be used to prepare the composition of the present invention.

Typically, the inventive process to prepare nanoparticle(s) or compositions is in accordance with the following specific disclosure.

Regarding step (a), the nanoparticle core is formed through the chemical reduction of the metal salt using the reducing agent.

The molar ratio of reducing agent to metal salt in step (a) is typically from about 1:1 to about 1:50. Preferably, the molar ratio of reducing agent to metal salt in step (a) is from about 1:1 to about 1:25, more preferably from about 1:1 to about 1:20, more preferably from about 1:2 to about 1:10, more preferably from about 1:2 to about 1:7, more preferably from about 1:2 to about 1:5. A particularly preferred molar ratio of reducing agent to metal salt is about 1:3.

The temperature for the reaction in step (a) is typically from about 20 °C to about 160 °C. Preferably, the temperature for the reaction in step (a) is from about 40 °C to about 150 °C, more preferably about 50 °C to about 145 °C, more preferably about 60 °C to about 140 °C, more preferably about 80 °C to about 120 °C, more preferably about 90 °C to about 110 °C. A particularly preferred temperature for the reaction in step (a) is about 100 °C.

The incubation time for the reducing agent and metal salt in step (a) - that is, the time in which the reducing agent and metal salt are boiled together - is typically between about 10 minutes to about 120 minutes. Preferably, incubation time for the reducing agent and metal salt in step (a) is between about 20 minutes to about 110 minutes, more preferably about 30 minutes to about 90 minutes, more preferably about 40 minutes to about 80 minutes, more preferably about 50 minutes to about 70 minutes. A particularly preferred incubation time for the reducing agent and metal salt in step (a) is about 60 minutes.

Typically, following incubation of the reducing agent and metal salt, heat is removed and the reaction mixture is allowed to return to room temperature (~20 °C) under constant stirring.

The total duration of step (a) is therefore typically between about 10 minutes to about 24 hours. Preferably, the total duration of step (a) is between about 20 minutes to about 20 hours, more preferably about 30 minutes to about 18 hours, more preferably about 60 minutes to about 18 hours. One preferred total duration of step (a) is about 2 hours.

The stirring speed of the reaction mixture in step (a) is typically from about 200 rpm to about 1500 rpm. Preferably, the stirring speed of the reaction mixture in step (a) is from about 200 rpm to about 1200 rpm, more preferably about 300 rpm to about 1000 rpm, more preferably about 300 rpm to about 700 rpm. A particularly preferred stirring speed of the reaction mixture in step (a) is about 500 rpm.

The metal salt may be any metal salt of a chemical element having an atomic number of from about 44 to about 83. Exemplary metal salts include chloroauric acid (HAuCl₄), silver nitrate (AgNO₃), silver acetate (CH₃COOAg), silver perchlorate (AgClO₄), potassium tetrachloroplatinate (K₂PtCl₄), platinum (IV) chloride (PtCl₂), and hexachloroplatinic acid (H₂PtCl₆). When the core comprises gold, the metal salt is preferably HAuCl₄. Preferably, the core does comprise gold.

The nature of the reducing agent is not particularly limited, and includes plant, fungal and bacterial extracts in addition to conventional chemical reducing agents. Suitable reducing agents can be readily selected by the skilled person based on their common general knowledge for a specific metal salt. Exemplary reducing agents include trisodium citrate (Na₃C₆H₅O₇), hydrazine (N₂H₄), ascorbic acid (C₆H₈O₆), sodium borohydride (NaBH₄), and exopolysaccharides, and exopolysaccharides in combination with L-ascorbic acid. Of these, trisodium citrate is typically preferred, especially when the core comprises gold. Trisodium citrate has the added advantage of acting as a dual reducing and capping agent. Many other reducing agents may require the use of an additional capping agent to prevent nanoparticle agglomeration. A capping agent is an agent that is functionalised onto the nanoparticle surface to improve colloidal stability. It does not function as a reducing agent *per se,* although certain agents (such as trisodium citrate) may be able to fulfil both these roles. The skilled person can readily determine whether the use of a capping agent, in addition to the reducing agent, is desirable. Suitable such capping agents in this regard include trisodium citrate, the polyethylene glycol (PEG), polyethyleneimine, poly(N-(2-hydroxypropyl) methacrylamide, chitosan, ethylenediaminetetraacetic acid (EDTA), polyvinyl pyrrolidone (PVP), polyvinyl alcohol (PVA), and bovine serum albumin (BSA).

In a particularly preferred embodiment, the metal salt is HAuCl₄ and the reducing agent is trisodium citrate. As trisodium citrate is used as the reducing agent, no additional / separate capping agent is required. This produces a nanoparticle with a gold core.

Regarding step (b), the stabilising / targeting agent precursors refer to any species that is converted to a stabilising / targeting agent as defined above upon reaction with, and attachment to, the unfunctionalised nanoparticle in step (b). For instance, the precursor could be a salt or ester of the stabilising / targeting agent, or stabilising / targeting agent with any other leaving group. Similarly, the targeting and/or stabilising agent may be charged, and the precursor thereof is the neutral form, or vice versa.

Preferably, the stabilising and targeting agents are added directly in step (b), i.e. no precursors are used.

Optionally, the stabilising agent or precursor thereof and/or targeting agent or precursor thereof are solubilised in a solvent prior to addition to the dispersion in step (b). Any suitable solvents can be used in this regard. Typically, the targeting agent (or precursor) is solubilised but the stabilising agent (or precursor) is not. The solvent used is typically acetonitrile or a solvent containing acetonitrile. When used, the solvent typically has a maximum concentration in the step (b) reaction mixture of 0.5 %v/v. For instance, the stabilising agent or precursor thereof and/or targeting agent or precursor thereof may be solubilised in a 1:1 (v/v) to 1: 10 (v/v) acetonitrile and water solution. A preferred solvent in this regard is a 1:4 (v/v) acetonitrile and water solution (i.e. 20% v/v).

If a solvent is used in this regard, then typically the process comprises a further step, step (c), wherein the "as synthesised" batch of nanoparticles is centrifuged following completion of step (b) to remove up to about 50% v/v, preferably up to about 60% v/v, more preferably up to about 70% v/v, more preferably up to about 80% v/v, more preferably up to about 90% v/v of the synthesis media. Particularly preferably, the "as synthesised" batch of nanoparticles is centrifuged following completion of step (b) to remove about 95% v/v of the synthesis media. Advantageously, this centrifugation step can also be used to remove any unbound stabilising agent or precursor thereof and/or targeting agent or precursor thereof. Preferably, step (c) is used in the process of the invention, and said step is also used to remove unbound stabilising agent or precursor thereof and/or targeting agent or precursor thereof.

If carried out, step (c) typically takes place between step (b) and step (d). In other words, following centrifugation, the removed synthesis media is typically replaced with water, preferably ultrapure water, to obtain a formulation containing the nanoparticles of the invention. This formulation typically comprises a maximum concentration of acetonitrile (or other non-water solvent if that is used instead of acetonitrile) of about <0.0025% v/v.

The molar ratio of stabilising agent or precursor thereof and targeting agent or precursor thereof added at step (b) is preferably from about 1:2 to about 1:4, more preferably about 1:3.

The order in which the stabilising and targeting agents are added to the dispersion in step (b) is not particularly limited. For example, they can be added sequentially or simultaneously. If added sequentially, the stabilising agent can be added first, followed by the targeting agent. Alternatively, the targeting agent can be added first, followed by the stabilising agent. Typically, the stabilising agent is added first, followed by the targeting agent.

The incubation time for the stabilising agent or precursor thereof and targeting agent or precursor thereof in step (b) is typically between about 10 minutes to about 24 hours. Preferably, the incubation time for the stabilising agent or precursor thereof and targeting agent or precursor thereof in step (b) is between about 30 minutes to about 20 hours, more preferably about 1 hour to about 15 hours, more preferably about 2 hours to about 10 hours, more preferably about 3 hours to about 9 hours, more preferably about 4 hours to about 8 hours. A particularly preferred incubation time for the stabilising agent or precursor thereof and targeting agent or precursor thereof in step (b) is about 6 hours.

The total duration of step (b) is typically between about 10 minutes to about 24 hours. Preferably, the total duration of step (b) is between about 30 minutes to about 20 hours, more preferably about 1 hour to about 15 hours, more preferably about 2 hours to about 10 hours, more preferably about 3 hours to about 9 hours, more preferably about 4 hours to about 8 hours. A particularly preferred total duration of step (b) is about 6 hours.

The temperature for the reaction in step (b) is typically from about 2 °C to about 35 °C. Preferably, the temperature for the reaction in step (b) is from about 5 °C to about 30 °C, more preferably about 10 °C to about 30 °C, more preferably about 15 °C to about 25 °C. A particularly preferred temperature for the reaction in step (b) is about 20 °C.

The stirring speed of the reaction mixture in step (b) is typically from about 200 rpm to about 1500 rpm. Preferably, the stirring speed of the reaction mixture in step (a) is from about 200 rpm to about 1200 rpm, more preferably about 300 rpm to about 1000 rpm, more preferably about 300 rpm to about 700 rpm. A particularly preferred stirring speed of the reaction mixture in step (a) is about 500 rpm.

### Methods of treatment, diagnosis and imaging

The present invention is also directed to methods of treatment, diagnosis and imaging, and to nanoparticles and compositions for use in these methods.

In particular, the present invention includes a method of treating cancer comprising administering a nanoparticle or composition of the invention to a subject having cancer.

The present invention also includes a nanoparticle or composition for use in the treatment of cancer, the treatment comprising administering the nanoparticle or composition to a subject having cancer, wherein the nanoparticle and composition are according to the invention.

Both of the above treatments typically further comprise the use of ionising radiation radiotherapy to irradiate the site of the cancer within the subject following administration of the nanoparticle or composition to the subject. Preferably, the ionising radiation is X-ray radiation, γ-ray photon beam radiation, electron beam radiation, positron beam radiation, proton beam radiation, radioisotope emission radiation, or a combination thereof.

The present invention is also directed to an *in vitro* method for killing or attenuating the growth of a cancer cell, the method comprising:
- treating the cancer cell with a nanoparticle of the invention or a composition of the invention, and then
- irradiating the cancer cell with ionising radiation.

The present invention is also directed to an *ex vivo* method for killing or attenuating the growth of a cancer cell, the method comprising:
- treating the cancer cell with a nanoparticle of the invention or a composition of the invention, and then
- irradiating the cancer cell with ionising radiation.

Again, the ionising radiation is preferably X-ray radiation, γ-ray photon beam radiation, electron beam radiation, positron beam radiation, proton beam radiation, radioisotope emission radiation, or a combination thereof. The radiation typically comprises irradiating the site of the cancer with irradiation from an external source or from a radioactive material inside the patient or subject.

As used in any of the above methods / treatments, the energy of the ionising radiation is typically of about 2 kV to about 24 MV. The specific energy will depend on the type of treatment. The skilled person can readily determine suitable energy ranges in this regard. Typical ranges, though, are ionising radiation of about 2 kV to about 24 MV, preferably about 100 kV to about 20 MW, more preferably, about 500 kV to about 18 MV. For instance, the energy may be about 3 MV to about 18 MV, such as about 2 MV to about 8 MV, such as for example about 6 MV.

As used in any of the above methods / treatments, administration of the nanoparticle or composition is not particularly limited. Typically, though, administration is achieved via injection, preferably by injection directly into the site of cancer.

The cancer or cancer cell of any of the above methods or treatments is not particularly limited. Typically, though, the cancer is selected from prostate cancer, breast cancer and/or lung cancer, or cells thereof. Preferably, the cancer to be treated is prostate cancer. Similarly, the cancer cells to be killed or attenuated are preferably prostate cancer cells.

The present invention also includes a method of imaging a subject comprising administering a nanoparticle of the invention or a composition of the invention to a subject and then imaging the subject using an imaging device.

The present invention also includes a nanoparticle or composition for use in a method of diagnosis, wherein the nanoparticle and composition are according to the invention. Typically, the method comprises detecting the presence of cancer in a patient.

The imaging device to be used in the method of imaging / diagnosis is not particularly limited. The imaging devices typically employ one or more of the following techniques:
X-ray, computed tomography, magnetic resonance imaging, optical imaging, optical coherence tomography, positron emission tomography, and ultrasound.

In all of the treatments / methods described herein, the patient or subject to be treated is a human or animal, preferably a human. All of the nanoparticles and compositions are those of the invention, as defined further above.

### Mechanism of action

The primary aim of radiotherapy is to deliver a therapeutic dose to the tumour target, while sparing the normal healthy surrounding tissue from damage. Recent advances in external beam radiotherapy (EBRT) including SBRT (Stereotactic body radiation therapy) and advanced imaging techniques have greatly improved tumour targeting limiting off target tissue toxicity, however greater tumour control and radiotherapy precision may be achieved through the use of effective radiation enhancers, also known as radiosensitisers.

High atomic number chemical elements such as gold can act as potent radiosensitisers due to their high atomic number and mass energy absorption coefficient relative to soft tissue. In particular, high atomic number nanoparticles can absorb more energy per mass unit than soft tissue, leading to an increase in the dose deposition in the localised vicinity of the nanoparticle.

There are two main mechanisms of radiosensitisation.

*Physical mechanism of radiosensitisation:* the Compton and Photoelectric effects are the main mechanisms by which radiation interacts with high atomic number chemical elements such as gold. In the case of the photoelectric effect, an incident photon is absorbed by an electron, causing its ejection from the inner shell. Consequently, the vacancy left by the ejected electron can be filled by an outer shell electron falling into its place, as such the loss of energy can lead to the ejection of another outer shell electron or Auger electron in a process known as the `Auger cascade'. In this case, up to 10 electrons can be emitted from one atom following a single inner shell ionisation. Furthermore, due to their low energies they can result in a high dose deposition within tens of nanometres in the vicinity of the material. This effect is more pronounced when the chemical element is in the nanoparticle form as these electrons are more likely to escape into the local environment, as opposed to the bulk form where the low energy short range electrons are trapped within the material.

*Chemical and Biological mechanism of radiosensitisation:* chemical and biological responses also contribute to the radiosensitisation potential of the nanoparticles of the invention, including reactive oxygen species production, oxidative stress, DNA repair inhibition and cell cycle effects.

Inflammation is an enabling characteristic of the tumour microenvironment, influencing cell survival, proliferation and angiogenesis. Target receptors are activated in part by treatment-induced inflammatory response. For instance, radiation promotes expression of CXCR1/CXCR2, which in turn initiate downstream signalling (PI3K/Akt and MAPK/ERK), leading to angiogenesis, cell survival, invasion and metastasis.

G-protein coupled receptors (GPCR) are integral membrane proteins that function as cell surface receptors involved in both normal physiological and pathological processes. There are over 650 members of the GPCR superfamily (with approximately 400 of these identified as druggable targets). While there is high sequence conservation within the 7-transmembrane-domain (TM) of the core, there is high diversity in the cytoplasmic loops (i1, i2, and i3) and the c-terminal i4 domain among GPCRs. GPCRs can interact with a variety of ligands including (but not limited to) small molecules, lipids, peptides and proteins. When a ligand interacts with its GPCR it induces signal transduction across the cell membrane. This results in a conformational change in the receptor leading to changes downstream in cellular effector function. Signal transduction pathways can include canonical (heterotrimer G proteins) and non-canonical (i.e. signalosomes - composed of several intracellular proteins).

Radiotherapy primes tumours to overexpress the preferred targeting receptor for the nanoparticle of the invention.

### EXAMPLES

### Example 1 - nanoparticle synthesis

The following exemplary method illustrates how the nanoparticles of the invention can be synthesised in one specific approach in accordance with the preparation process of the present invention. The skilled person can readily adapt this specific synthesis using means known in the art and their common general knowledge in order to synthesise other nanoparticles of the invention.

A modified Turkevich-Frens methodology was used to produce 15 nm citrate capped gold nanoparticles. Sodium citrate reduction of a chloroauric acid (HAuCl₄) solution was used to obtain dispersed gold nanoparticles. The dispersed gold nanoparticles were then co-functionalised with 5 kDa PEG-thiol (the stabilising agent) and a pepducin corresponding to SEQ ID 3 (the targeting agent) in a 1:3 molar ratio of stabilising agent to targeting agent.

The following materials and equipment were used.

| **Product Name** | **Product number** | **Mn (g/mol)** |
|---|---|---|
| Gold (III) chloride trihydrate (≥49.0% Au) (supplied by Sigma-Aldrich) | G4022 | 393.833 |
| Sodium citrate tribasic dihydrate (supplied by Sigma-Aldrich) | S4641 | 294.1 |
| UltraPure distilled H₂O (UP-H₂O) (supplied by Thermo) | 10977 | 18.02 |
| *O*-[2-(3-Mercaptopropionylamino)ethyl]-*O*'-methylpolyethylene glycol (5000 Daltons) - PEG-Thiol (supplied by Sigma-Aldrich) | 11124 | 5000 |
| Pepducin corresponding to SEQ ID 3 | | 1950.46 |
| Acetonitrile (supplied by Sigma-Aldrich) | 271004 | 41.05 |
| Hydrochloric acid 32% (supplied by VWR) | 20254.321 | 36.46 |
| Nitric acid 69% (supplied by VWR) | 20425.420 | 63.01 |

| **Peptide characteristics** | |
|---|---|
| **Pepducin Name** | SEQ ID 3 |
| **Sequence (N to C)** | Pal-RTLFKAHMGQKHRC-NH₂ |
| **HPLC Purity** | >98% |
| **N-terminus** | Pal (Palmitoyl) |
| **C-terminus** | NH₂ (Amidation) |
| **Salt Form** | Acetate salt |
| **Solubility** | 1:4 (Acetonitirile:H₂O) |

| **Equipment name** | **Capacity** |
|---|---|
| Stirring/Heating Mantle | 500 ml |
| Reflux system (Round bottom flask and condenser) | 500 ml |
| Magnetic stirrer | 500 ml |
| Balance | 0.1 mg - 220 g |
| Glass bottles | 500 ml |
| Magnetic flea (stirring bar) | 8 mm × 25 mm |
| Pipettes and tips | 10 µl - 1 ml |
| Centrifuge tubes | 50 ml |
| Serological pipette controller | 5 - 25 ml |

**Synthesis of unfunctionalised nanoparticle (step (a)):** The synthesis of the gold nanoparticle core was based on a modified Turkevich-Frens methodology (Turkevich J, Stevenson PC, Hillier J. A study of the nucleation and growth processes in the synthesis of colloidal gold. Discussions of the Faraday Society. 1951;11(0):55-75. Frens G. Controlled Nucleation for the Regulation of the Particle Size in Monodisperse Gold Suspensions. Nature Physical Science. 1973;241(105):20-2.). Prior to the gold nanoparticle synthesis, all glassware was cleaned with aqua regia (nitric acid/hydrochloric acid, 1:3), rinsed with UP-H₂O and left to dry. A 0.254 mM aqueous solution of gold (III) chloride trihydrate was prepared in 400 ml of UP-H₂O in a round bottom flask and brought to a boil under reflux with continuous stirring. The solution was allowed to boil for 15 minutes then 9 ml of a 1% wt/v solution of sodium citrate tribasic dihydrate was added (final concentration: 0.77 mM). The solution was allowed to boil under reflux and continuous stirring for 1 hour. After 1 hour, the flask was left to cool to room temperature under continuous stirring leading to the formation of 15 nm citrate capped gold nanoparticle (unfunctionalised).

| **Example:** 400 ml batch synthesis, unfunctionalised nanoparticles | |
|---|---|
| **Chloroauric acid (HAuCl₄)** | 0.25 mM |
| **Trisodium citrate** | 0.77 mM |
| **Final size** (TEM) | 15 nm |
| **Molar Ratio Au:Citrate** | 1:3 |
| **Concentration** | 188 µM (∼40 µg/ml) |

**Nanoparticle functionalisation (step (b)):** 2.14 mg of PEG-thiol was reconstituted in 1 ml of UP-H₂O by vortex mixing (30 seconds). PEG-thiol was added dropwise to the unfunctionalised nanoparticle dispersion at a final concentration of 5.34 µg/ml and stirred (500 rpm) at room temperature overnight. For purification, the reaction mixture was transferred to centrifuge tubes and centrifuged at 13000 g for 1.5 hours to remove unbound PEG. Following centrifugation AuNP-PEG was redispersed in UP-H₂O. 2.5 mg of pepducin corresponding to SEQ ID 3 was reconstituted in 1 ml of acetonitrile/water (1:4, v/v) by hand mixing (30 seconds). Co-functionalisation was achieved by adding pepducin corresponding to SEQ ID 3 dropwise to the AuNP-PEG dispersion at a final concentration of 6.25 µg/ml and stirred (500 rpm) at room temperature overnight. For purification, the reaction mixture was transferred to centrifuge tubes and centrifuged at 13000 g for 1.5 hours to remove unbound targeting agent. Following centrifugation, the nanoparticles were redispersed in UP-H₂O.

| **Example:** 400 ml batch synthesis, functionalised nanoparticles | µg/ml | Molarity |
|---|---|---|
| **Au** | 40 | 188 µM |
| **SH-PEG** - 5000 g/mol | 5.34 | 1 µM |
| **Pepducin** corresponding to SEQ ID 3 (Pal-RTLFKAHMGQKHRC-NH2) - 1950.5 g/mol | 6.25 | 3 µM |
| **Molar** Ratio PEG:Pepducin | | 1:3 |

The resultant nanoparticles had a shelf life of 2 months (4 °C).

### Example 2 - alternative nanoparticle formulations

The following exemplary alternative nanoparticle formulations were also prepared using similar methods according to the present invention.

| **Targeting agent** | | | | |
|---|---|---|---|---|
| **Pepducin** | **GPCR intracellular loop** | **Amino acid sequence** | **Receptor** | **Charge** |
| SEQ ID 3 | i3 | SEQ ID 3 | CXCR1/2 | Positive |

| **Alternative stabilising agent : targeting agent molar ratio** | | |
|---|---|---|
| **Molar ratio (PEG:pepducin)** | **PEG (µg/mL)** | **Pepducin (µg/mL)** |
| 1:1 | 10.68 | 4.17 |
| 1:3 | 5.34 | 6.25 |
| 1:9 | 2.14 | 7.50 |

| **Alternative stabilising agent M_{w}** | | |
|---|---|---|
| **M_{w} (g/mol)** | **PEG (µg/mL)** | **Molar ratio (PEG:pepducin)** |
| 800 | 1.71; 1.37; 1.03; 0.85 | 1:1; 1:1.5; 1:2.3; 1:3 |
| 2000 | 4.27; 3.42; 2.56; 2.14 | 1:1; 1:1.5; 1:2.3; 1:3 |
| 5000 | 5.34; 4.27; 3.20; 2.14 | 1:1; 1:3; 1:4; 1:6; 1:9 |

| **Alternative stabilising agent** | | | |
|---|---|---|---|
| **Polymer** | **Structure** | **M_{w} (g/mol)** | **Charge** |
| Polyethylene glycol | Hydrophilic polymer | 5000 | Neutral |
| Chitosan | Polysaccharide | 5000 | Positive |
| Branched polyethyleneimine modified with multiple poly(ethylene glycol)-thiols | Hydrophilic polymer | 2000 (polyethyleneimine); | positive |
| | | 5000 (polyethylene glycol) | |
| Poly(N-(2-hydroxypropyl)methacrylamide | Hydrophilic polymer - macromolecule carrier | 29700 | neutral |
| | | 36500 | |

### Example 3 - nanoparticle characterisation

Four different gold nanoparticle core sizes were evaluated, with the results shown in Figure 1a. Starting in the top left panel of Figure 1a and moving clockwise, these had hydrodynamic diameters of 49 nm, 29 nm, 17 nm and 15 nm respectively, and were synthesised using 0.27, 0.54, 0.82, 1.28 mM sodium citrate reducing agent respectively. DLS measurements showed a decrease in size as the amount of sodium citrate was increased (Figure 1b). Zeta potential (Figure 1c) of all four nanoparticles was strongly negative at -35.5, -37.86, -20.43 and -23.57 mV respectively. This is due to the binding of citric acid which confers an overall negative charge to the surface of the nanoparticle. UV/Vis spectroscopy provided further evidence of the change in nanoparticle size, with maximal plasmon resonance peaks at 534, 528, 520 and 520 nm respectively (Figure 1d).

Extensive characterisation of bare and functionalised nanoparticles has been performed, including the nanoparticles of Examples 1 and 2. The results are provided in the Figures, including for the formulation obtained in Example 1 (Figure 2), and the alternative formulations of Example 2, such as using different PEG:pepducin molar ratios (Figure 4), alternative PEG molecular weights (Figures 5 and 6), and alternative stabilising agents including poly(N-(2-hydroxypropyl) methacrylamide (Figure 7), polyethyleneimine (Figure 8) and chitosan (Figure 9). In the Example 1 formulation, DLS measurements showed an increase in size from bare citrate capped nanoparticles following the addition of PEG, with a further increase in size following pepducin attachment (Figure 2b). The addition of pepducin did not significantly alter the PDI when compared with pegylated nanoparticles, thus confirming good colloidal stability (Figure 2c). Conjugation of the pepducin to the pegylated nanoparticles also resulted in a rapid shift to a positive surface charge (Figure 2d). UV/Vis provided further evidence of successful PEG and pepducin attachment resulting in a 2.5 and 4.1 nm shift in λ-max from AuNP-Citrate (Figure 2e). Absolute confirmation of PEG and pepducin conjugation was obtained using FTIR (Figure 2f). Characteristic vibrations found in ethylene glycol moieties are expressed at 1080-1019 cm⁻¹ (C-O-C stretching). Prominent differences were seen in the visible spectra for both pegylated nanoparticles and the fully functionalised nanoparticle preparing in Example 1. The presence of a mixed monolayer PEG/pepducin arrangement was confirmed by the additional peaks assigned as follows: amide band A at 3407 cm⁻¹ (NH stretching), amide band I peak at 1614 cm⁻¹ (NH bending and C=O stretching) amide band II at 1382 cm⁻¹ (NH bending and C-H stretching), and N-H wagging at 615 cm⁻¹.

### Example 4 - nanoparticle stability

The nanoparticles of the invention have been shown to enjoy robust stability, especially under extreme and physiological conditions.

Serum and salt stability assays were performed using DLS over a 24 h period on all nanoparticle formulations. While the unfunctionalised nanoparticles (citrate capped) aggregated instantly following incubation with a physiologically relevant salt solution (0.15 M NaCl) as seen by the significant increase in size and PDI, neither the pegylated nanoparticles nor the nanoparticles prepared in Example 1 exhibited any significant change in size or PDI following the 24 h incubation in 0.15 M NaCl (Figure 3a). Furthermore, a significant increase in size of the unfunctionalised nanoparticles followed incubation in RPMI containing 10% serum, indicating non-specific protein absorption (Figure 3b). Importantly, neither the pegylated nanoparticles nor the nanoparticles prepared in Example 1 exhibited any significant change in size or PDI over a 24 h incubation period in either salt or 10% serum. To more closely simulate the *in vivo* blood plasma conditions both the pegylated nanoparticles nor the nanoparticles prepared in Example 1 were incubated in RPMI containing 40% serum. Again, both preparations remained stable in 40% serum over a 24 h period and showed no significant change in either size or PDI demonstrating high levels of stability (Figure 3c). Alternative PEG:pepducin molar ratios did not improve stability in 0.15 M, with both 30:70 and 40:60 increasing in size following exposure to salt for 24 hr (Figure 6). Importantly, preparations synthesized with the alternative stabilising polymers poly(N-(2-hydroxypropyl) methacrylamide (Figure 7) and polyethylenimine (Figure 8) demonstrated high levels of stability, while some agglomeration was observed for those synthesised with chitosan following exposure to physiologically relevant salt conditions (Figure 9).

### Example 5 - nanoparticle internalisation in cancer cells and target specificity

The nanoparticles of the invention have been shown to enjoy excellent internalisation.

Visualisation of nanoparticle accumulation in prostate cancer tumour cells using hyperspectral microscopy (red overlay) clearly shows that conjugation of the targeting agent (pepducin corresponding to SEQ ID 3) delivers superior internalisation over both unfunctionalised (citrate capped) nanoparticles and nanoparticles functionalised with only a stabilising agent (PEG) (Figure 10a, b; Figure 11a, b); quantification by ICP-MS revealed a >300-fold increase in nanoparticle accumulation in prostate cancer cells over the pegylated nanoparticles (Figure 10c, d; Figure 11c). An alternative 1:1 stabilising agent to targeting agent (PEG:pepducin) molar ratio attenuated this internalisation, but the presence of the targeting agent still delivered greater internalisation as compared against the pegylated nanoparticle (Figure 12). Other nanoparticles of the invention with alternative formulations, such as gold nanoparticles co-functionalised with pepducin and poly(N-(2-hydroxypropyl) methacrylamide (Figure 13) or polyethylenimine (Figure 14) were also readily internalised in tumour cells. Figure 15 (Example 1 formulation) and Figure 16 (alternative formulation with poly(N-(2-hydroxypropyl) methacrylamide and polyethylenimine) clearly demonstrate the target receptor (CXCR1/2) specificity of the nanoparticles of the invention, with high quantities of the Example 1 nanoparticles internalised within 2 h of treatment (see middle row). This was in contrast to the unfunctionalised nanoparticles, but importantly also against cells pre-treated (2 h) with free pepducin corresponding to SEQ ID 3 in order to block the receptor binding site of the nanoparticles. In this setting, nanoparticle internalisation was severely attenuated. Furthermore, in the absence of radiation, intracellular nanoparticles of Example 1 were shown to not impact the long-term viability of the cells (Figure 17). This clearly shows that the nanoparticles of the invention are not cytotoxic.

### Example 6 - cancer cell sensitisation using nanoparticles

The nanoparticles of the invention have been shown to enhance cancer cell sensitivity to radiotherapy at kV and MV energies, specifically with respect to prostate cancer cells. It has also been demonstrated that the nanoparticles of the invention enjoy a synergistic interaction in this regard, specifically with respect to the combination of stabilising and targeting agents that co-functionalise the nanoparticle core.

Treatment with the Example 1 formulation resulted in a 26%, 29% and 50% enhancement in sensitivity (sensitiser enhancement ratio) to radiotherapy in DU145, sh11.02 and PC3 prostate cancer cells respectively (Figure 18a). Conversely, treatment with 800 nM of pepducin corresponding to SEQ ID 3 as a single-agent produced a small but nonsignificant dose enhancement (Figure 18b). Importantly, the nanoparticles of the invention more than doubled PC3 cell sensitivity to radiotherapy (2.2 fold) at doses of 2.5 Gy, in comparison to pegylated-nanoparticles (no targeting agent) (0.9 fold) and pepducin-alone (1.2 fold) (Figure 18c). Hence, it can be seen that the combination of targeting and stabilising agents on the nanoparticle results in a synergistic interaction that enhances radiosensitivity to an extent greater than either agent alone. Conversely, alternative 1:1 stabilising agent to targeting agent (PEG:pepducin) molar ratio resulted in no additional radiosenitisation benefit in either DU145 or PC3 cells (Figure 19). Radioenhancement was observed in C42B prostate cancer cells following treatment with the Example 1 nanoparticles (Figure 20). Additionally, >50% increase in PC3 cell sensitivity to radiotherapy was achieved with nanoparticles formulated with the alternative stabilising polymer poly(N-(2-hydroxypropyl) methacrylamide (Figure 21). Importantly, treatment with the Example 1 nanoparticles resulted in a 26% and 14% enhancement in sensitivity to radiotherapy in DU145 cells (Figure 22a) and PC3 cells (Figure 22b) at clinical radiotherapy (MV) energies in prostate cancer cell lines.

### Example 7 - tumour sensitisation using nanoparticles

The nanoparticles of the invention have also been shown to enhance prostate tumour sensitivity to radiotherapy.

Injection of nanoparticles prepared in accordance with Example 1 (three doses at 8 mg/kg - total pepducin dose ~ 105 µg per average 28 g mouse) directly into established PC3 prostate tumours (intratumoural) in combination with clinically relevant doses of radiation (2 x 2.5 Gy) inhibited tumour growth by approximately 20% (p=0.036), prolonging time to experimental endpoint over radiation-only (Figure 25). Computational modelling of tumour response to radiotherapy incorporating a 20% dose enhancement to simulated CHHiP trial treatment plans illustrates the potential impact an effective radiosensitiser could have over existing standard of care treatment. In Figure 26, PC3 prostate tumour bearing animals were injected with a single intratumoural implant of Example 1 nanoparticles at 25 mg/kg (green line) or 50 mg/kg (purple line) (Figure 26b). At these concentrations, tumours were exposed to a maximum gold dose of 0.7 mg and 109 µg pepducin (25 mg/ml), or double this for 50 mg/kg treated animals. After 24 h, the nanoparticles were activated using a single dose of X-ray radiation (2.5 Gy) delivered in contraparallel beams using the Xstrahl small animal radiation research platform (SARRP). This setup minimises entry and exit beam dose to normal tissue, thus limiting radiation induced damage, more accurately replicating clinical external beam radiotherapy treatment plans. Mean tumour volume data are presented in Figure 26c for untreated control tumours, verses radiation alone or AuX2R at two concentrations. Unsurprisingly, by day 14 radiation alone significantly (p<0.01) attenuated tumour growth over untreated control tumour (Figure 26d). However, a single infusion of inventive nanoparticles (irrespective of treatment concentration) further attenuated tumour growth over radiation alone. Given that experimental endpoint is defined by tumours exceeding 400 mm³, no animals (minimum of n=7, maximum of n=8 per group) assigned to the nanoparticle treated groups reached this point by day 63, compared to all radiation only treated tumours that had reached experimental end (Figure 26e).

### Example 8 - imaging using nanoparticles

The nanoparticles of the invention have also been shown to improve imaging methods, specifically with respect to improved CT contrast.

CBCT imaging of phantoms loaded with nanoparticles prepared in accordance with Example 1 were obtained at 60 kV (Figure 23a), 120 kV (Figure 23b), 40 kV and 50 kV (Figure 23c), with the nanoparticles showing obvious contrast enhancement relative to water. CT values were measured for ROI within a slice of the phantom and reported as Hounsfield Units (HU); calibrated by assigning the values 0 HU to water and -1000 HU to air. Typical HU for prostate (+40 HU) are presented in Figure 23c in comparison to the inventive nanoparticles (60 kV; +300 HU at 4mg/ml), suggesting that the nanoparticle formulation has the potential to act as an efficient contrast agent. This was further verified *in vivo* as CBCT imaging following intratumoural injection of nanoparticles prepared in accordance with Example 1 *in vivo* showed an increase in contrast in the tumour area (Figure 24). Taken together with the *in vitro* data, this shows that the nanoparticles of the invention act as an efficient contrast agent, improving prostate tumour delineation on CT.

### Example 9 - cell viability of PCa and DU145cell lines following exposure to pepducins

Alamar blue viability assays assessed the impact of inhibiting CXCL8 pro-inflammatory signalling on the viability of various PCa cell lines (Figure 28). Three increasing concentrations of pepducin (150 nM, 300 nM and 600 nM) were assayed following a 6 h incubation. Following exposure to pepducin cells were washed in PBS and 10% Alamar blue solution added for 4 h. Fluorescence was then measured at 540 - 570 nm. Treatment with either, A) comparative pepducin (corresponding to SEQ ID 6), B) inventive pepducin or C) scrambled control (corresponding to SEQ ID 7), did not significantly reduce cell viability over a period of 6 h. (N = 4 +/- SEM).

While no significant reduction was observed in PC3 cells (see Figure 28 A), the comparative pepducin caused a maximal 25% reduction in viability at 300 nM and the inventive pepducin resulted in no significant reduction in viability with only an 8% reduction at 300 nM. The scrambled control produced no significant affects to PC3 cells (see Figure 28 C).

In DU145 and DU145 isogenic cell lines, the impact of CXCL8 attenuation was more pronounced than that seen in PC3 cells where treatment with both 300 nM and 600 nM comparative pepducin resulted in a significant reduction in viability (see Figure 29 D & G), reflecting the importance of CXCL8 signalling on the viability of these cells lines.

Following exposure to pepducins, cells were washed in PBS and 10% Alamar blue solution added for 4 h. Fluorescence was then measured at 540 - 570 nm. Inhibition of CXCL8 signalling by the inventive pepducin and the scrambled control did not show any significant toxicity in DU145 parental cells (B and C), NT01 (E and F) or sh1102 cells (H and I). By contrast, the comparative pepducin significantly reduced cell viability at both 300 nM and 600 nM in both DU145 parental cells (A) and isogenic cells (D and G). (N=4 +/- SEM).

A consistent reduction in viability was observed using the inventive pepducin. This confirms that the addition of cysteine does not significantly alter the bioactivity of the pepducin. The direct impact of CXCL8 antagonism was moderately reduced.

## Claims

1. A nanoparticle comprising a core, wherein the core comprises a chemical element having an atomic number of from about 44 to about 83, and wherein the core is functionalised with a stabilising agent and a targeting agent.

2. The nanoparticle of claim 1, wherein the targeting agent is not RME peptide (CKKKKKKSEDEYPYVPN) or H5WYG peptide (GCGLFHAIAHFIHGGWHGHHGWYG).

3. The nanoparticle of any preceding claim, wherein:
(i) the core comprises a chemical element selected from gold, platinum, iridium, osmium, bismuth, tantalum, thulium, hafnium and gadolinium, optionally wherein the core consists of gold, platinum, iridium, osmium, an oxide of bismuth, an oxide of tantalum, an oxide of thulium, an oxide of hafnium, or an oxide of gadolinium; and/or
(ii) the core comprises elemental gold, optionally wherein the core consists of elemental gold; and/or
(iii) the core is a solid core, optionally wherein the core is a solid core that is spherical or approximately spherical in shape; and/or
(iv) the core comprises an additional chemical element in addition to the chemical element having an atomic number of from 44 to about 83, for instance a magnetic chemical element such as iron.

4. The nanoparticle of any preceding claim, wherein the core has a mean hydrodynamic diameter of from about 5 to about 50 nm.

5. The nanoparticle of claim 4, wherein the mean hydrodynamic diameter is determined using dynamic light scattering in accordance with ISO 22412:2017.

6. The nanoparticle of any preceding claim, wherein the nanoparticle is obtainable by combining the core with:
(i) the stabilising agent and the targeting agent, or
(ii) a stabilising agent precursor and the targeting agent, or
(iii) the stabilising agent and a targeting agent precursor, or
(iv) a stabilising agent precursor and a targeting agent precursor,
wherein the stabilising agent, or precursor thereof, and the targeting agent, or precursor thereof, are added respectively at a molar ratio of from about 1:2 to about 1:5, optionally from about 1:2 to about 1:4.

7. The nanoparticle of any preceding claim, wherein the stabilising agent and targeting agent are independently attached to the core via a coordinate covalent bond, a covalent bond, or an ionic bond.

8. The nanoparticle of any preceding claim, wherein the stabilising agent enhances the stability of the nanoparticle, optionally wherein the stabilising agent reduces agglomeration and deterioration, especially under biological or physiological conditions.

9. The nanoparticle of any preceding claim, wherein the stabilising agent comprises a natural polymer, synthetic polymer, biocompatible polymer, biomedical polymer, hydrophilic polymer, or a combination thereof, optionally wherein the natural polymer, synthetic polymer, biocompatible polymer, biomedical polymer, and/or hydrophilic polymer is attached to the core via a coordinate covalent bond.

10. The nanoparticle of any preceding claim, wherein the stabilising agent comprises polyethylene glycol, polyethyleneimine, poly(N-(2-hydroxypropyl) methacrylamide, chitosan, or a combination thereof, optionally wherein the polyethylene glycol, polyethyleneimine, poly(N-(2-hydroxypropyl) methacrylamide, and/or chitosan is attached to the core via a coordinate covalent bond.

11. The nanoparticle of any preceding claim, wherein the stabilising agent comprises polyethylene glycol, and/or has a weight average molecular weight of from about 800 to about 10000 g/mol, preferably from about 3000 to about 7000 g/mol.

12. The nanoparticle of any preceding claim, wherein the stabilising agent is thiol-terminated.

13. The nanoparticle of any preceding claim, wherein the targeting agent targets cells expressing chemokine receptors, optionally wherein (i) the targeting agent enables cellular uptake of the nanoparticle in cells expressing chemokine receptors, and/or (ii) the chemokine receptors are selected from CXCR1, CXCR2, and combinations thereof.

14. The nanoparticle of any preceding claim, wherein the targeting agent comprises a peptide and/or small molecule, optionally wherein:
(i) the peptide is a pepducin, protein, polypeptide, oligopeptide, aptamer, antibody, and/or antibody fragment, and/or
(ii) the small molecule is a small molecule inhibitor, such as a small molecule inhibitor that targets CXCR1 or CXCR2.

15. The nanoparticle of any preceding claim, wherein the targeting agent comprises a peptide, preferably a pepducin or a protein, optionally wherein the targeting agent is a peptide.

16. The nanoparticle of any preceding claim, wherein the targeting agent comprises a pepducin comprising a peptide chain and an N-terminal lipid moiety, wherein the peptide chain comprises fewer than about 50 amino acid residues and has a C-terminal cysteine residue, optionally wherein:
(i) the peptide chain is derived from the amino acid sequence of an intracellular loop domain of a G protein coupled receptor; and/or
(ii) the peptide chain is derived from the amino acid sequence of a chemokine receptor; and/or
(iii) the peptide chain is derived from the amino acid sequence of CXCR1 or CXCR2; and/or
(iv) the peptide chain comprises an amino acid sequence having at least about 80% sequence identity to SEQ ID 1; and/or
(v) the N-terminal lipid moiety is a C10-25 lipid moiety, preferably a moiety derived from a C10-C25 fatty acid, more preferably a moiety derived from palmitic acid, myristic acid, stearic acid or lithocholic acid, even more preferably a palmitoyl moiety, and/or
(vi) the C-terminal cysteine residue is amidated.

17. The nanoparticle of any preceding claim, wherein the targeting agent comprises a pepducin comprising an N-terminal palmitoyl moiety and a C-terminal cysteine residue, wherein the pepducin has a peptide chain having at least about 80% sequence identity to SEQ ID 1, and wherein the pepducin comprises fewer than about 50 amino acid residues, optionally wherein the targeting agent comprises a pepducin corresponding to SEQ ID 2 or SEQ ID 3.

18. The nanoparticle of any preceding claim, wherein:
- the core comprises elemental gold,
- the stabilising agent comprises a polyethylene glycol that is attached to the core via a coordinate covalent bond, and
- the targeting agent comprises a pepducin that is attached to the core via a coordinate covalent bond.

19. The nanoparticle of any preceding claim, wherein:
- the core comprises elemental gold,
- the stabilising agent comprises a thiol-terminated polyethylene glycol, and
- the targeting agent comprises a pepducin comprising an N-terminal palmitoyl moiety and a C-terminal cysteine residue, wherein the pepducin has a peptide chain having at least about 80% sequence identity to SEQ ID 1 and the pepducin comprises fewer than about 50 amino acid residues, optionally wherein the targeting agent comprises a pepducin corresponding to SEQ ID 2 or SEQ ID 3.

20. A nanoparticle consisting of an elemental gold core that is functionalised with a thiol-terminated polyethylene glycol and a pepducin comprising an N-terminal palmitoyl moiety and a C-terminal cysteine residue, wherein the pepducin has a peptide chain having at least about 90% sequence identity to SEQ ID 1 and comprises fewer than about 50 amino acid residues, optionally wherein the pepducin corresponds to SEQ ID 2 or SEQ ID 3.

21. A composition comprising one or more nanoparticle(s) according to any preceding claim, optionally wherein the composition is a colloid solution.

22. The composition of claim 21, wherein the composition is a pharmaceutical composition that comprises a therapeutically effective amount of the nanoparticle(s) and a pharmaceutically acceptable excipient.

23. The composition of claim 21, wherein the composition is a diagnostic composition.

24. The composition of any one of claims 21 to 23, wherein the concentration of the nanoparticle(s) in the composition is about 20 µg/L to about 200 g/L.

25. A kit comprising:
- one or more nanoparticle(s) according to any one of claims 1 to 20 or a composition according to any one of claims 21 to 24; and
- means for administering the nanoparticle(s) or composition to a subject.

26. A pepducin comprising a peptide chain and an N-terminal lipid moiety derived from a C10-C25 fatty acid, wherein the peptide chain has an N-terminal arginine residue and a C-terminal cysteine residue, and the peptide chain has at least about 90% sequence identity to SEQ ID 1, and wherein the pepducin comprises fewer than about 50 amino acid residues, optionally wherein (i) the C10-C25 fatty acid is selected from palmitic acid, myristic acid, stearic acid and lithocholic acid ; and/or (ii) the pepducin corresponds to SEQ ID 2 or SEQ ID 3; and/or (iii) the pepducin corresponds to SEQ ID 3.

27. A process for preparing a nanoparticle, said process comprising:
(a) combining a metal salt with a reducing agent to obtain a dispersion comprising an unfunctionalised nanoparticle, wherein the metal salt comprises a chemical element having an atomic number of from about 44 to about 83; and
(b) to the dispersion obtained in step (a), adding a stabilising agent or precursor thereof and a targeting agent or precursor thereof at a molar ratio of from about 1:2 to about 1:5 respectively to obtain a nanoparticle functionalised with both a stabilising agent and a targeting agent;
optionally wherein the process further comprises dispersing the functionalised nanoparticle obtained in step (b) in water, an aqueous solution or a buffer to create a colloid solution.

28. The process of claim 27, wherein the nanoparticle is a nanoparticle according to any one of claims 1 to 20.

29. A method of treating cancer comprising administering a nanoparticle according to any one of claims 1 to 20 or a composition according to any one of claims 21 to 24 to a subject having cancer.

30. The method of claim 29, further comprising the use of ionising radiation radiotherapy to irradiate the site of the cancer within the subject following administration of the nanoparticle or composition to the subject, optionally wherein the ionising radiation is X-ray radiation, γ-ray photon beam radiation, electron beam radiation, positron beam radiation, proton beam radiation, radioisotope emission radiation, or a combination thereof.

31. A method of imaging a subject comprising administering a nanoparticle according to any one of claims 1 to 19 or a composition according to any one of claims 20 to 23 to a subject and then imaging the subject using an imaging device.

32. A nanoparticle according to any one of claims 1 to 20 or a composition according to any one of claims 21 to 24 for use in the treatment of cancer, the treatment comprising administering the nanoparticle or composition to a subject having cancer.

33. A nanoparticle according to any one of claims 1 to 20 or a composition according to one of claims 21 to 24 for use in a method of diagnosis, optionally wherein the method comprises detecting the presence of cancer in a patient.

34. An *in vitro* method for killing or attenuating the growth of a cancer cell, the method comprising:
- treating the cancer cell with a nanoparticle according to any one of claims 1 to 20 or a composition according to claim 21, 22 or 24, and then
- irradiating the cancer cell with ionising radiation.

35. The method of any one of claims 29, 30 or 34 or the nanoparticle or composition for use of claim 32 or 33, wherein the cancer is selected from prostate cancer, breast cancer and/or lung cancer, preferably wherein the cancer is prostate cancer.

## Amended claims

### Amended claims in accordance with Rule 137(2) EPC.

1. A nanoparticle comprising a core, wherein the core comprises gold, and wherein
- the core is functionalised with a stabilising agent and a targeting agent,
- the stabilising agent comprises a natural polymer, synthetic polymer, biocompatible polymer, biomedical polymer, hydrophilic polymer, or a combination thereof; and
- the targeting agent targets cells expressing chemokine receptors.

2. The nanoparticle of claim 1, wherein the targeting agent is not RME peptide (CKKKKKKSEDEYPYVPN) or H5WYG peptide (GCGLFHAIAHFIHGGWHGHHGWYG).

3. The nanoparticle of any preceding claim, wherein:
(i) the core further comprises a chemical element selected from platinum, iridium, osmium, bismuth, tantalum, thulium, hafnium and gadolinium; and/or
(ii) the core comprises elemental gold, optionally wherein the core consists of elemental gold; and/or
(iii) the core is a solid core, optionally wherein the core is a solid core that is spherical or approximately spherical in shape; and/or
(iv) the core comprises an additional chemical element in addition to gold, for instance a magnetic chemical element such as iron.

4. The nanoparticle of any preceding claim, wherein the core has a mean hydrodynamic diameter of from about 5 to about 50 nm.

5. The nanoparticle of claim 4, wherein the mean hydrodynamic diameter is determined using dynamic light scattering in accordance with ISO 22412:2017.

6. The nanoparticle of any preceding claim, wherein the nanoparticle is obtainable by combining the core with:
(i) the stabilising agent and the targeting agent, or
(ii) a stabilising agent precursor and the targeting agent, or
(iii) the stabilising agent and a targeting agent precursor, or
(iv) a stabilising agent precursor and a targeting agent precursor,
wherein the stabilising agent, or precursor thereof, and the targeting agent, or precursor thereof, are added respectively at a molar ratio of from about 1:2 to about 1:5, optionally from about 1:2 to about 1:4.

7. The nanoparticle of any preceding claim, wherein the stabilising agent and targeting agent are independently attached to the core via a coordinate covalent bond, a covalent bond, or an ionic bond.

8. The nanoparticle of any preceding claim, wherein the stabilising agent enhances the stability of the nanoparticle, optionally wherein the stabilising agent reduces agglomeration and deterioration, especially under biological or physiological conditions.

9. The nanoparticle of any preceding claim, wherein the natural polymer, synthetic polymer, biocompatible polymer, biomedical polymer, and/or hydrophilic polymer is attached to the core via a coordinate covalent bond.

10. The nanoparticle of any preceding claim, wherein the stabilising agent comprises polyethylene glycol, polyethyleneimine, poly(N-(2-hydroxypropyl) methacrylamide, chitosan, or a combination thereof, optionally wherein the polyethylene glycol, polyethyleneimine, poly(N-(2-hydroxypropyl) methacrylamide, and/or chitosan is attached to the core via a coordinate covalent bond.

11. The nanoparticle of any preceding claim, wherein the stabilising agent comprises polyethylene glycol, and/or has a weight average molecular weight of from about 800 to about 10000 g/mol, preferably from about 3000 to about 7000 g/mol.

12. The nanoparticle of any preceding claim, wherein the stabilising agent is thiol-terminated.

13. The nanoparticle of any preceding claim, wherein the targeting agent enables cellular uptake of the nanoparticle in cells expressing chemokine receptors.

14. The nanoparticle of any preceding claim, wherein the chemokine receptors are selected from CXCR1, CXCR2, and combinations thereof.

15. The nanoparticle of any preceding claim, wherein the targeting agent comprises a peptide and/or small molecule, optionally wherein:
(i) the peptide is a pepducin, protein, polypeptide, oligopeptide, aptamer, antibody, and/or antibody fragment, and/or
(ii) the small molecule is a small molecule inhibitor, such as a small molecule inhibitor that targets CXCR1 or CXCR2.

16. The nanoparticle of any preceding claim, wherein the targeting agent comprises a peptide, optionally wherein the targeting agent is a peptide.

17. The nanoparticle of any preceding claim, wherein the targeting agent comprises a pepducin or a protein.

18. The nanoparticle of any preceding claim, wherein the targeting agent comprises a pepducin comprising a peptide chain and an N-terminal lipid moiety, wherein the peptide chain comprises fewer than about 50 amino acid residues and has a C-terminal cysteine residue, optionally wherein:
(i) the peptide chain is derived from the amino acid sequence of an intracellular loop domain of a G protein coupled receptor; and/or
(ii) the peptide chain is derived from the amino acid sequence of a chemokine receptor; and/or
(iii) the peptide chain is derived from the amino acid sequence of CXCR1 or CXCR2; and/or
(iv) the peptide chain comprises an amino acid sequence having at least about 80% sequence identity to SEQ ID 1; and/or
(v) the N-terminal lipid moiety is a C10-25 lipid moiety, preferably a moiety derived from a C10-C25 fatty acid, more preferably a moiety derived from palmitic acid, myristic acid, stearic acid or lithocholic acid, even more preferably a palmitoyl moiety, and/or
(vi) the C-terminal cysteine residue is amidated.

19. The nanoparticle of any preceding claim, wherein the targeting agent comprises a pepducin comprising an N-terminal palmitoyl moiety and a C-terminal cysteine residue, wherein the pepducin has a peptide chain having at least about 80% sequence identity to SEQ ID 1, and wherein the pepducin comprises fewer than about 50 amino acid residues, optionally wherein the targeting agent comprises a pepducin corresponding to SEQ ID 2 or SEQ ID 3.

20. The nanoparticle of any preceding claim, wherein:
- the core comprises elemental gold,
- the stabilising agent comprises a polyethylene glycol that is attached to the core via a coordinate covalent bond, and
- the targeting agent comprises a pepducin that is attached to the core via a coordinate covalent bond.

21. The nanoparticle of any preceding claim, wherein:
- the core comprises elemental gold,
- the stabilising agent comprises a thiol-terminated polyethylene glycol, and
- the targeting agent comprises a pepducin comprising an N-terminal palmitoyl moiety and a C-terminal cysteine residue, wherein the pepducin has a peptide chain having at least about 80% sequence identity to SEQ ID 1 and the pepducin comprises fewer than about 50 amino acid residues, optionally wherein the targeting agent comprises a pepducin corresponding to SEQ ID 2 or SEQ ID 3.

22. A nanoparticle consisting of an elemental gold core that is functionalised with a thiol-terminated polyethylene glycol and a pepducin comprising an N-terminal palmitoyl moiety and a C-terminal cysteine residue, wherein the pepducin has a peptide chain having at least about 90% sequence identity to SEQ ID 1 and comprises fewer than about 50 amino acid residues, optionally wherein the pepducin corresponds to SEQ ID 2 or SEQ ID 3.

23. A composition comprising one or more nanoparticle(s) according to any preceding claim, optionally wherein the composition is a colloid solution.

24. The composition of claim 23, wherein the composition is a pharmaceutical composition that comprises a therapeutically effective amount of the nanoparticle(s) and a pharmaceutically acceptable excipient.

25. The composition of claim 23, wherein the composition is a diagnostic composition.

26. The composition of any one of claims 23 to 25, wherein the concentration of the nanoparticle(s) in the composition is about 20 µg/L to about 200 g/L.

27. A kit comprising:
- one or more nanoparticle(s) according to any one of claims 1 to 22 or a composition according to any one of claims 23 to 26; and
- means for administering the nanoparticle(s) or composition to a subject.

28. A pepducin comprising a peptide chain and an N-terminal lipid moiety derived from a C10-C25 fatty acid, wherein the peptide chain has an N-terminal arginine residue and a C-terminal cysteine residue, and the peptide chain has at least about 90% sequence identity to SEQ ID 1, and wherein the pepducin comprises fewer than about 50 amino acid residues, optionally wherein (i) the C10-C25 fatty acid is selected from palmitic acid, myristic acid, stearic acid and lithocholic acid ; and/or (ii) the pepducin corresponds to SEQ ID 2 or SEQ ID 3; and/or (iii) the pepducin corresponds to SEQ ID 3.

29. A process for preparing a nanoparticle, said process comprising:
(a) combining a metal salt with a reducing agent to obtain a dispersion comprising an unfunctionalised nanoparticle, wherein the metal salt comprises gold; and
(b) to the dispersion obtained in step (a), adding a stabilising agent or precursor thereof and a targeting agent or precursor thereof at a molar ratio of from about 1:2 to about 1:5 respectively to obtain a nanoparticle functionalised with both a stabilising agent and a targeting agent, wherein the targeting agent targets cells expressing chemokine receptors and the stabilising agent comprises a natural polymer, synthetic polymer, biocompatible polymer, biomedical polymer, hydrophilic polymer, or a combination thereof;
optionally wherein the process further comprises dispersing the functionalised nanoparticle obtained in step (b) in water, an aqueous solution or a buffer to create a colloid solution.

30. The process of claim 29, wherein the nanoparticle is a nanoparticle according to any one of claims 1 to 22.

31. A method of imaging a subject comprising administering a nanoparticle according to any one of claims 1 to 22 or a composition according to any one of claims 23 to 26 to a subject and then imaging the subject using an imaging device.

32. A nanoparticle according to any one of claims 1 to 22 or a composition according to any one of claims 23 to 26 for use in the treatment of cancer, the treatment comprising administering the nanoparticle or composition to a subject having cancer.

33. A nanoparticle according to any one of claims 1 to 22 or a composition according to one of claims 23 to 26 for use in a method of diagnosis, optionally wherein the method comprises detecting the presence of cancer in a patient.

34. An *in vitro* method for killing or attenuating the growth of a cancer cell, the method comprising:
- treating the cancer cell with a nanoparticle according to any one of claims 1 to 22 or a composition according to claim 23, 24 or 26, and then
- irradiating the cancer cell with ionising radiation.

35. The method of claim 34 or the nanoparticle or composition for use of claim 32 or 33, wherein the cancer is selected from prostate cancer, breast cancer and/or lung cancer, preferably wherein the cancer is prostate cancer.
